(19) Europäisches Patentamt
European Patent Office
Office européen des brevets

(11) EP 0 398 230 B1

(12) EUROPEAN PATENT SPECIFICATION

(45) Date of publication and mention of the grant of the patent:
**21.01.1998 Bulletin 1998/04**

(51) Int Cl.[6]: **C07D 473/30**, C07H 19/173, C07D 405/04, A61K 31/70, A61K 31/52, A61K 31/505

(21) Application number: **90109065.4**

(22) Date of filing: **14.05.1990**

(54) **Antiviral, highly water soluble, stable, crystalline salts of 2',3'-dideoxyinoside monohydrate, 2',3'-dideoxy-2',3'-didehydrothymidine monohydrate and 2',3'-dideoxy-2'-fluoroinosine hemihydrate**

Antivirale, gut wasserlösliche, stabile, kristalline Salze von 2',3'-Dideoxyinosinmonohydrat, 2',3'-Dideoxy-2',3'-didehydrothymidinmonohydrat und 2',3'-Dideoxy-2'-fluoroinosinhemihydrat

Sels cristallins, stables, antiviraux et bien hydrosolubles de 2',3'-didéoxyinosine monohydrate, 2',3'-didéoxy-2',3'-didehydrothymidine monohydrate et 2',3'-didéoxy-2'-fluoroinosine hémihydrate

(84) Designated Contracting States:
**AT BE CH DE DK ES FR GB GR IT LI LU NL SE**

(30) Priority: **15.05.1989 US 352065**
**01.05.1990 US 514261**

(43) Date of publication of application:
**22.11.1990 Bulletin 1990/47**

(73) Proprietor: **Bristol-Myers Squibb Company**
**New York, N.Y. 10154 (US)**

(72) Inventors:
- **Kaplan, Murray Arthur**
  **Syracuse, N.Y. 13290 (US)**
- **Perrone, Robert Kevin**
  **Liverpool, New York 13090 (US)**
- **Bogardus, Joseph Ballard**
  **Syracuse, N.Y. 13104 (US)**

(74) Representative: **Kinzebach, Werner, Dr. et al**
**Patentanwälte**
**Reitstötter, Kinzebach und Partner**
**Postfach 86 06 49**
**81633 München (DE)**

(56) References cited:
**EP-A- 0 216 510**
**EP-A- 0 273 277**
**EP-A- 0 287 313**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### BACKGROUND OF THE INVENTION

#### Field of the Invention

The present invention concerns highly water soluble, stable, crystalline antiviral (including antiretroviral) sodium salts of 2',3'-dideoxyinosine, 2',3'-dideoxy-2',3'-didehydrothymidine and 2'3'-dideoxy-2' -fluoroinosine.

#### Background Information

Infectious viral diseases are recognized as an important medical problem. Progress against infectious viral disease requires the development of drugs with selective antiviral activity, while remaining benign to normal cell lines. A number of antiviral agents currently under study which seem to possess some selectivity are nucleoside analogs. In general, these compounds are structural analogs of the naturally occurring nucleosides. Structural modification in either the purine or pyrimidine base nucleus and/or the saccharide component results in a synthetically modified nucleoside derivative which, when incorporated into a viral nucleic acid forming process, acts to disrupt further synthesis of viral nucleic acid.

Effectiveness of these antiviral agents depends on selective conversion by viral enzymes, but not by host enzymes, to the corresponding nucleotide analog which is then converted to the triphosphate followed by incorporation into viral nucleic acid. A problem with this antiviral strategy has been the emergence of certain viral strains whose enzymes poorly promote phosphorylation of the nucleoside analogs. To circumvent this problem, intact nucleotide analogs appear to be potentially quite useful as antivirals for incorporation into viral nucleic acid.

EP-A-216 510 discloses the use of 2',3'-dideoxyinosine, 2',3'-dideoxyguanosine or 2', 3'-dideoxyadenosine for alleviating the cytopathic effects of HIV on cells.

PCT application W087/01284 to Mitsuya and Broder describes the use of 2',3'-dideoxyinosine for use against AIDS.

EP 273,277 to Lin and Prusoff discloses the use of 2',3'-dideoxy-2',3'-didehydrothymidine in treating patients infected with a retrovirus.

Erik De Clercq, "Potential Drugs for the Treatment of AIDS", Journal of Antimicrobial Chemotherapy, 23, Suppl. A, 35-46, (1989), describes the use of dideoxynucleoside analogues to inhibit the infectivity and cytopathic effect of human immunodeficiency virus (HIV).

EP 287,313 discloses 2',3'-dideoxy-2'-fluoroinosine (F-ddI) and its activity against HIV.

### SUMMARY OF THE INVENTION

The present invention concerns very highly water soluble, stable, crystalline sodium salts of 2',3'-dideoxy-2',3'-didehydrothymidine ("D4T") of the formula

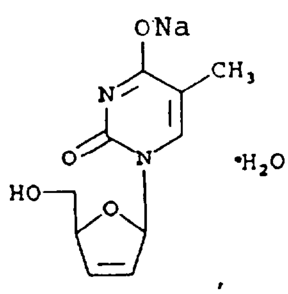

(I)

,

2´,3´-dideoxyinosine ("DDI")

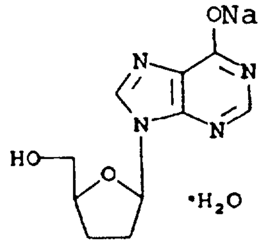

(II)

and 2',3'-dideoxy-2'-fluoroinosine (F-ddI)

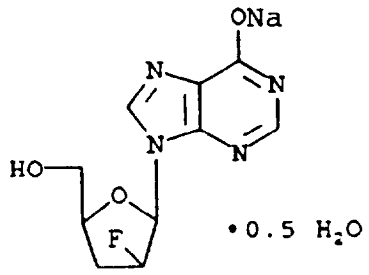

(III)

The X-ray spectrum of D4T is shown in Fig. 8 and that of DDI and F-ddl are given in examples 1 and 3.

The present invention also concerns pharmaceutical compositions comprising an antiviral effective amount of such salts and a solid, liquid or gaseous physiologically acceptable diluent.

Still further, the present invention relates to the use of said salts for preparing a pharmaceutical composition for treating a virus infection in a warm blooded animal, for example, a human.

## BRIEF DESCRIPTION OF THE DRAWINGS

Fig. 1 depicts an IR for sodium-2',3'-dideoxyinosine.
Fig. 2 depicts a TGA for sodium-2',3'-dideoxyinosine.
Fig. 3 depicts a DSC for sodium-2',3'-dideoxyinosine.
Fig. 4 depicts a NMR for sodium-2',3'-dideoxyinosine.
Fig. 5 depicts an IR for sodium-2',3'-dideoxy-2',3'-didehydrothymidine.
Fig. 6 depicts a TGA for sodium-2',3'-dideoxy-2',3'-didehydrothymidine.
Fig. 7 depicts a DSC for sodium-2',3'-dideoxy-2',3'-didehydrothymidine.
Fig. 8 depicts X-ray data for sodium-2',3'-dideoxy-2'3'-didehydrothymidine.
Fig. 9 depicts an NMR for sodium-2',3'-dideoxy-2',3'-didehydrothymidine.
Fig. 10 depicts an IR for sodium-2',3'-dideoxy-2'-fluoroinosine.
Fig. 11 depicts an NMR for sodium-2',3'-dideoxy-2'-fluoroinosine.
Fig. 12 depicts a DSC for sodium-2,3-dideoxy-2-fluoroinosine.
Fig. 13 depicts a TGA for sodium-2',3'-dideoxy-2'-fluoroinosine.

## DETAILED DESCRIPTION OF THE INVENTION

The compounds of this invention have desirable antiviral activity. They exhibit activity against viruses, for example, Herpes Simplex virus I, Herpes Simplex virus II, cytomegalovirus, Varicella Zoster virus, influenza virus, vaccinia, polio, rubella, smallpox, cowpox, Epstein-Barr virus, measles virus, human respiratory virus, papillomavirus and Sinbis virus, just to mention a few and also against retroviruses, for example, virus of human immunodeficiency (HIV).

As mentioned above, the compounds of the present invention are useful active ingredients in human and veterinary medicine for the treatment and prophylaxis of diseases caused by retroviruses. Examples of fields of indication in human medicine regarding retroviruses are as follows:

(1) the treatment or prophylaxis of human retrovirus infections;

(2) the treatment or prophylaxis of diseases caused by HIV (virus of human immunodeficiency; previously called HTLV III/LAV or AIDS) and the stages associated therewith such as ARC (AIDS related complex) and LAS (lymph adenopathy syndrome) and the immune weakness and encephalopathy caused by this retrovirus;

(3) the treatment or prophylaxis of HTLV I infection or HTLV II infection;

(4) the treatment or prophylaxis of the AIDS carrier state (AIDS transmitter state); and

(5) the treatment or prophylaxis of diseases caused by hepatitis B virus.

Examples of indications in veterinary medicine are as follows:

(1) Maedivisna (in sheep and goats),

(2) progressive pneumonia virus (PPV) (in sheep and goats),

(3) caprine arthritis encephalitis virus (in sheep and goats),

(4) Zwoegerziekte virus (in sheep),

(5) infectious virus of anemia (of the horse), and

(6) infections caused by cat leukemia virus.

For use against viral infections the compounds of this invention can be formulated into pharmaceutical preparations. Such preparations are composed of one or more of the inventive compounds in association with pharmaceutically acceptable carriers. The reference Remington's Pharmaceutical Sciences, 17th Edition, A.R. Gennaro, editor (Mack Publishing Company, 1985) discloses typical carriers and methods of preparation.

The compounds of the invention are administered systemically to warm blooded animals, e.g., humans. By systemic administration is intended oral, rectal, and parenteral (i.e., intramuscular, intravenous, subcutaneous and nasal) routes. Generally, it will be found that when a compound of the present invention is administered orally, a larger quantity of the reactive agent may be required to produce the same effect as the smaller quantity given parenterally. In accordance with good clinical practice, it is preferred to administer the instant compounds at a concentration level that will produce an effective antiviral effect without causing any harmful or untoward side effects.

Therapeutically and prophylactically the instant compounds are usually given as pharmaceutical compositions comprised of an effective antiviral amount of a compound according to the invention and one or more pharmaceutically acceptable carriers, as stated hereinabove. Pharmaceutical compositions for effecting such treatment will contain a major or minor amount, e.g., from 100 to 0.5% of at least one compound of the present invention in combination with a pharmaceutical carrier, the carrier comprising one or more solid, semi-solid, or liquid diluents, fillers and formulation adjuvants which are non-toxic, inert and pharmaceutically acceptable.

Such pharmaceutical compositions are preferably in dosage unit form; i.e., physically discrete units containing a predetermined amount of the drug corresponding to a fraction or multiple of the dose which is calculated to produce the desired therapeutic response. Other therapeutic agents can also be present.

Pharmaceutical compositions providing from about 50 mg to 2 grams of the active ingredient per unit dose are preferred and are conventionally prepared as tablets, lozenges, capsules, powders, granules, aqueous or oily suspensions, syrups, elixirs, and aqueous solutions. Preferred oral compositions are in the form of tablets or capsules and may contain conventional excipients such as binding agents (e.g., syrup, acacia, gelatin,sorbitol, tragacanth or polyvinylpyrrolidone), fillers (e.g., lactose, sugar, corn starch, calcium phosphate, sorbitol, or glycine), lubricants (e.g., magnesium stearate, talc, polyethylene glycol or silica), disintegrants (e.g., starch), buffering agents (inorganic or organic) and wetting agents (e.g., sodium lauryl sulfate). Solutions or suspensions of an inventive compound with conventional pharmaceutical vehicles are employed for parenteral compositions, such as an aqueous solution for intravenous injection or an oily suspension for intramuscular injection. Such compositions having the desired clarity, stability and adaptability for parenteral use are obtained by dissolving from 0.1% to 35% by weight of an active inventive compound in water or a vehicle comprising a polyhydric aliphatic alcohol such as glycerine, propylene glycol, and polyethylene glycol or mixtures thereof. The polyethylene glycols comprise a mixture of non-volatile, usually liquid, polyethylene glycols which are soluble in both water and organic liquids and have molecular weights from about 200 to 1500.

The inventive compounds are prone to acid hydrolysis. For optimum bioavailability, tablets, capsules and granules required for oral dosage should be enteric coated, strongly buffered against gastric acid, or both.

The salts of this patent application are stable, crystalline and of very high water solubility (> 300 mg/ml), ideally suited for high concentration, low volume IV-IM injectables. Most importantly, the high alkalinity (pH 9.5-11) of these new and novel salts allow for self-buffering against gastric acid. Thus, less buffer could be required in oral dosage forms.

The salts of the invention are reversibly "immobilized" in the enolic form, and thus, can be considered as intermediates for synthesis of varied potentially bio-active derivatives as enol esters (prodrugs) and enol ethers. The esters and ethers could also function as protecting (blocking) groups, allowing for synthetic work on other functions or moieties of the molecules.

Considering the biological activities possessed by the compounds of the instant invention, it can be seen that these compounds have antiviral properties, particularly suited to their use in combating viral infections. Thus, another aspect of the instant invention concerns a process for treating viral (including retroviral) infections in a mammal in need of such treatment which comprises systemic administration to such mammal of an effective dose of an inventive compound.

On the basis of testing, an effective dose could be expected to be from about 0.1 to about 5 mg/kg body weight with about 1 to about 4 mg/kg body weight a preferred dosage range. It is envisioned that for clinical antiviral application, compounds of the instant invention will be administered in the same manner as for the reference drug zidorudine (AZT). For clinical applications, however, the dosage and dosage regimen must in each case be carefully adjusted, utilizing sound professional judgement and consideration of the age, weight and condition of the recipient, the route of administration and the nature and gravity of the illness. Generally a daily oral dose will comprise from about 150 mg to about 5 grams, preferably 50-1500 mg of an inventive compound administered from one to three times a day. In some instances, a sufficient therapeutic effect can be obtained at lower doses, while in others, larger doses will be required.

## Description of the Specific Embodiments

The compounds which constitute this invention and their methods of preparation will appear more fully from a consideration of the following examples which are given for the purpose of illustration. All temperatures are understood to be in degrees C when not specified. All compounds gave satisfactory elemental analyses.

### Example 1

### Preparation of Sodium-DDI Monohydrate

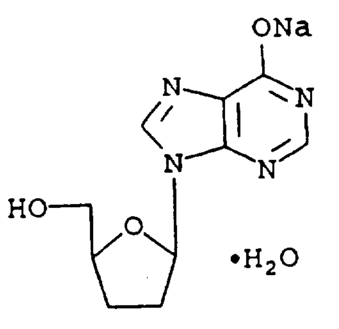

MW: DDI = 236.14
MW: NaOH = 40

$$\frac{1\ \text{g DDI}}{236.14} = \frac{X}{40} = \frac{169\ \text{mg NaOH}}{40} = 4.3\ \text{ml of 1N-NaOH is equivalent to 1 g of DDI for a 1/1 molar ratio.}$$

1. Add 1 g of DDI to 4.51 ml of aqueous IN-NaOH (1.05 molar equivalents with moderate stirring at 10-25°C over a 5 minute interval. A solution (pH 9.5 - 11), or near solution is obtained.

2. If required, pass the solution through the equivalent of a 0.2 - 0.45 micron Gelman HT-Tuffryn filter to clarify.

Steps 1 and 2 should be completed within 1.5 hours.

3. Add the Na-DDI solution, over a 10-15 minute interval to 50-75 ml of very vigorously stirred isopropanol (alternatively, acetone could be used). Crystals form. Continue vigorous stirring (closed system) for 2 hours.

4. Collect the crystals via a suitable vacuum filtration procedure. Do not draw excess air through the filter-cake. Tamp the crystalline filter-cake (under vacuum) to remove any cracks or fissures which may form. Do not draw excess air through the cake.

5. Wash the filter-cake with three separate 15 ml portions of isopropanol and then with three separate 20 ml portions of acetone. Do not draw excess air through the filter-cake between washings. Remove any cracks or fissures which may form between washings by tamping.

6. High vacuum dry the crystals at 24-35°C for 24 hours. Expected yield: 1-1.1g.

7. If desired, acetone can be totally substituted for the preferred isopropanol and the modes of addition reversed, if desired viz, the solvents added to the stirring aqueous DDI solution over a half-hour interval.

KOH and/or other strong organic and inorganic bases can be substituted for NaOH to form the corresponding salts.

Chemical and Physical Properties Obtained for Na-DDI·$H_2O$

1. Water Solubility: >300 mg/ml (pH 9.5-11)

2. Aqueous Solution Stability: 5% loss for 1 week at 50°C; 10% loss for 1 week at 70°C

3. Solid State Stability: No loss for 5 weeks at 70°C

4. Elemental Analysis: $C_{10}H_{11}N_4O_3Na \cdot H_2O$ ($C_{10}H_{13}N_4O_4Na$) MW - 276.26

| | Theory | Found |
|---|---|---|
| % C | 43.5 | 43.18 |
| % H | 4.7 | 4.6 |
| % N | 20.3 | 20.16 |
| % Na (Ash) | 8.3 | 8.26 |
| % $H_2O$ KF | 6.5 (for monohydrate) | 6.57 |

5. IR: See Fig. 1

6. TGA: See Fig. 2

7. DSC: See Fig. 3

8. X-Ray (film):

## X-Ray d-Lines

DDI

| d | | I/Io |
|---|---|---|
| 14.57 | - | 100 |
| 8.39 | - | 80 |
| 7.46 | - | 20 |
| 6.60 | - | 10 |
| 6.00 | - | 20 |

Na-DDI $H_2O$

| d | | I/Io |
|---|---|---|
| 8.75 | - | 60 |
| 7.82 | - | 80 |
| 6.21 | - | 30 |
| 5.81 | - | 20 |
| 5.28 | - | 10 |

DDI

| d | | I/Io |
|---|---|---|
| 5.50 | - | 20 |
| 5.14 | - | 20 |
| 4.79 | - | 30 |
| 4.46 | - | 30 |
| 3.54 | - | 90 |
| 3.21 | - | 40 |
| 3.04 | - | 30 |

Na-DDI $H_2O$

| d | | I/Io |
|---|---|---|
| 5.00 | - | 10 |
| 4.65 | - | 40 |
| 4.22 | - | 50 |
| 3.89 | - | 100 |
| 3.49 | - | 20 |
| 3.25 | - | 20 |
| 3.09 | - | 10 |
| 2.92 | - | 50 |
| 2.77 | - | 10 |
| 2.61 | - | 20 |

9. NMR: See Fig. 4 (no solvents present)

Table I

| HPLC Assay Method | | |
|---|---|---|
| Column: | IBM Phenyl, 5 micron, 4.5 x 150 nm | |
| Mobile Phase: | 98% 0.015M $NH_4H_2PO_4$ in Milli-Q Water, pH 7.1 with concentrated $NH_4OH$/2% acetonitrile | |
| Flow Rate: | 0.1 mg/ml in water | |
| Approximate Retention Times: | ddI: | 10 minutes |
| | Hypoxanthine: | 3 minutes |

Example 2

Preparation of Sodium-$D_4T$ Hydrate

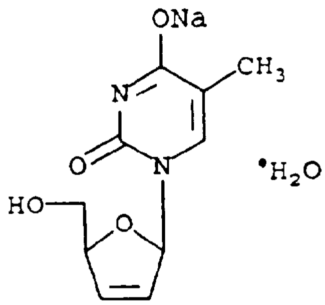

MW: $D_4T$ = 224.2
MW: NaOH = 40

$$\frac{1\text{ g D4T}}{224.21} = \frac{X}{40} = \frac{178.4\text{ mg NaOH}}{40}$$ = 4.46 ml of 1N-NaOH is equivalent to 1 g of $D_4T$ for a 1/1 molar ratio.

1. Add 1 g of $D_4T$ to 4.7 ml of aqueous IN-NaOH (1.05 molar equivalents) with moderate stirring at 15-25°C over a five minute interval. A solution (pH 9.5 - 11) or near solution is obtained.
2. Repeat and follow Steps 2-7 as described in Example 1 for the preparation of Na-DDI $H_2O$.
3. Expected yield of $D_4T{\cdot}H_2O$ = 1 - 1.1 g.

Chemical and Physical Parameters Obtained for Na-$D_4T{\cdot}H_2O$

1. Water Solubility: > 300 mg/ml (pH 9.5 - 11)
2. Aqueous Solution Stability: 1 week at 50°C-20% remaining
3. Solid State Stability: No loss for 1 week at 70°C
4. Elemental Analysis: $C_{10}H_{11}N_2O_4Na{\cdot}H_2O$ ($C_{10}H_{13}N_2O_5Na$) MW: 264.22

| | Theory | Found |
|---|---|---|
| % C | 45.5 | 45.05 |
| % H | 5.0 | 4.89 |
| % N | 10.6 | 10.37 |
| % Na | 8.7 | 8.43 |
| % $H_2O$ KF | 6.8 (for monohydrate) | 6.9 |

5. IR: See Fig. 5
6. TGA: See Fig. 6
7. DSC: See Fig. 7
8. X-Ray: See Fig. 8

| HPLC Assay Method | |
|---|---|
| Column | IBM Phenyl, 5 micron, 4.5 x 150 nm |
| Mobile Phase | 98% 0.015M $NH_4H_2PO_4$ in Milli-Q Water, pH 7.1 with concentrated $NH_4OH$/2% acetonitrile |
| Flow Rate | 0.1 ml/minute |
| Injection Volume | 25 microliter |

(continued)

| HPLC Assay Method | | |
|---|---|---|
| Run Time | 20 minutes | |
| Wavelength | 254 nm | |
| Temperature | ambient, 20-30°C | |
| Sample Conc. | approximately 0.1 mg/ml in water | |
| Approximate Retention Times | d4T | 8 minutes |
| | Thymine | 3 minutes |

Example 3

Preparation of Sodium F-ddI Hemihydrate

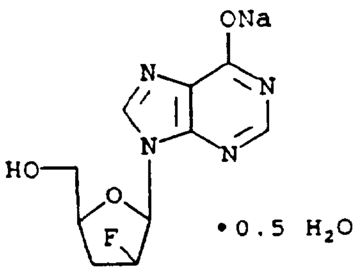

MW: FDDI = 254.24
MW: NaOH = 40

$$\frac{436\text{ mg FDDI}}{254.24} = \frac{x}{40} = \frac{68.6\text{ mg NaOH}}{40} = 1.72\text{ ml NaOH is equivalent to 436 mg of FDDI for a 1:1 molar ratio}$$

1. FDDI (426 mg) was slurried in 1.8 ml of 1N NaOH (256 mg/ml NaFDDI). A pH 9.9 solution was obtained in 2 minutes.

2. Two ml of isopropanol was added. The solution remained clear.

3. Isopropanol was slowly added with slurring. Heavy needle-like crystallization started when 15 ml of isopropanol was added. The mixture was slurried for 10 minutes.

4. Fifteen ml of acetone was added and the mixture was slurried an additional 15 minutes.

5. The crystals were removed by vacuum filtration, washed with 15 ml of acetone and 20 ml of ether, and vacuum-dried ($P_2O_5$ at 50°C for 2 hours and then at 24°C for 24 hours). Yield: 430 mg.

A 21 mg sample readily dissolved in 0.05 ml of water (≈ 400 mg/ml; pH 10.0).
Elemental Analysis: $C_{10}H_{10}N_4FO_3Na$

| | Theory | Found |
|---|---|---|
| % C | 42.0 | 41.19 |
| % H | 3.9 | 3.73 |
| % N | 19.6 | 18.8 |
| % F | 6.6 | 6.13 |
| % Na | 8.0 | 8.11 |
| % $H_2O$ KF | 3.23 (hemihydrate) | 3.23 |

X-Ray (film):

| X-Ray d-Lines | |
|---|---|
| d | I/Io |
| 16.20 | 50 |
| 9.40 | 50 |
| 6.91 | 50 |
| 5.81 | 50 |
| 5.40 | 100 |

NMR: See Fig. 11. Data consistent with proposed structure. No acetone or ether present. No α-isomer present.

IR: See Fig. 10.

TGA: See Fig. 13.

DSC: See Fig. 12.

**Claims**

**Claims for the following Contracting States : AT, BE, CH, LI, DE, DK, FR, GB, IT, LU, NL, SE**

**1.** Crystalline sodium 2',3'-dideoxy -2',3'-didehydrothymidine monohydrate of the formula

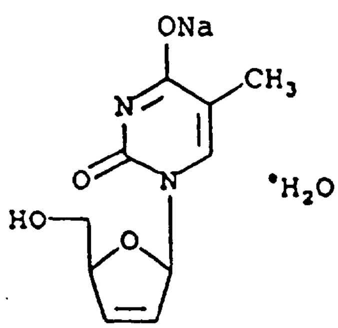

exhibiting an X-ray spectrum as shown in Figure 8.

**2.** Crystalline sodium 2',3'-dideoxyinosine monohydrate of the formula

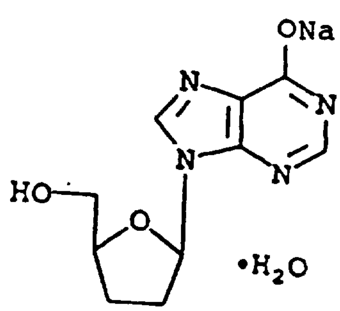

exhibiting the following X-ray d-lines:

| d | I/Io |
|---|---|
| 8.75 | 60 |
| 7.82 | 80 |
| 6.21 | 30 |
| 5.81 | 20 |
| 5.28 | 10 |
| 5.00 | 10 |
| 4.65 | 40 |
| 4.22 | 50 |
| 3.89 | 100 |

| d | I/Io |
|---|---|
| 3.49 | 20 |
| 3.25 | 20 |
| 3.09 | 10 |
| 2.92 | 50 |
| 2.77 | 10 |
| 2.61 | 20 |

**3.** Crystalline sodium 2',3'-dideoxy-2'-fluoroinosine hemihydrate of the formula

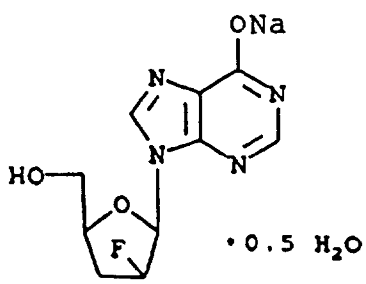

exhibiting the following X-ray d-lines:

| d | I/Io |
|---|---|
| 16.20 | 50 |
| 9.40 | 50 |
| 6.91 | 50 |
| 5.81 | 50 |
| 5.40 | 100 |

**4.** A pharmaceutical composition containing an antiviral effective amount of at least one salt according to claims 1 to 3 and a pharmaceutically acceptable carrier.

**5.** The composition of claim 4 in the form of a tablet, capsule or powder for oral administration enterically coated and buffered against gastric acid.

**6.** A process for preparing the composition of claim 4 or 5 which comprises providing an antiviral effective amount of at least one salt according to claims 1 to 3 in a pharmaceutically acceptable carrier in a form suitable for administration, preferably in the form of a tablet, capsule or powder for oral administration enterically coated and buffered against gastric acid.

7. The use of at least one salt according to anyone of claims 1 to 3 for preparing a pharmaceutical composition for treating a virus infection.

8. A process for the preparation of the crystalline salts according to claims 1 to 3, which comprises reacting 2',3'-dideoxy-2',3'-didehydrothymidine, 2',3'-dideoxyinosine or 2',3'-dideoxy-2'-fluoroinosine in an inert aqueous solvent system with an appropriate base providing the sodium cation and allowing the desired salt to crystallize from the solution.

**Claims for the following Contracting State : ES**

1. A process for the preparation of crystalline sodium 2',3'-dideoxy-2',3'-didehydrothymidine monohydrate of the formula

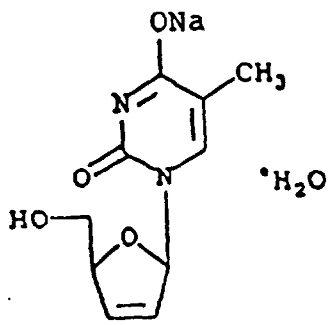

exhibiting an X-ray spectrum as shown in Figure 8, which comprises reacting 2',3'-dideoxy-2',3'-didehydrothymidine in an inert aqueous solvent system with an appropriate base providing the sodium cation and allowing the desired salt to crystallize from the solution.

2. A process for the preparation of crystalline sodium 2',3'-dideoxyinosine monohydrate of the formula

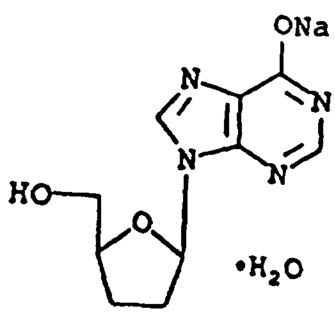

exhibiting the following X-ray d-lines:

| d | I/Io |
|---|---|
| 8.75 | 60 |
| 7.82 | 80 |
| 6.21 | 30 |
| 5.81 | 20 |
| 5.28 | 10 |
| 5.00 | 10 |
| 4.65 | 40 |
| 4.22 | 50 |
| 3.89 | 100 |

| d | I/Io |
|---|---|
| 3.49 | 20 |
| 3.25 | 20 |
| 3.09 | 10 |
| 2.92 | 50 |
| 2.77 | 10 |
| 2.61 | 20 |

which comprises reacting 2',3'-dideoxyinosine in an inert aqueous solvent system with an appropriate base providing the sodium cation and allowing the desired salt to crystallize from the solution.

3. A process for the preparation of crystalline sodium 2',3'-dideoxy-2'-fluoroinosine hemihydrate of the formula

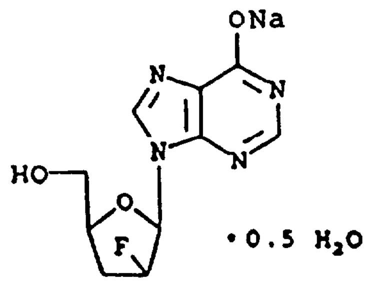

exhibiting the following X-ray d-lines:

| d | I/Io |
|---|---|
| 16.20 | 50 |
| 9.40 | 50 |
| 6.91 | 50 |
| 5.81 | 50 |
| 5.40 | 100 |

which comprises reacting 2',3'-dideoxy-2'-fluoroinosine in an inert aqueous solvent system with an appropriate base providing the sodium cation and allowing the desired salt to crystallize from the solution.

4. A process for preparing a pharmaceutical composition containing an antiviral effective amount of at least one salt as defined in claims 1 to 3 and a pharmaceutically acceptable carrier, which comprises providing an antiviral effective amount of at least one of said salts in a pharmaceutically acceptable carrier in a form suitable for administration.

5. A process according to claim 4 for preparing the composition in the form of a tablet, capsule or powder for oral administration enterically coated and buffered against gastric acid.

6. The use of at least one salt according to anyone of claims 1 to 3 for preparing a pharmaceutical composition for treating a virus infection.

**Claims for the following Contracting State : GR**

1. Crystalline sodium 2',3'-dideoxy-2',3'-didehydrothymidine monohydrate of the formula

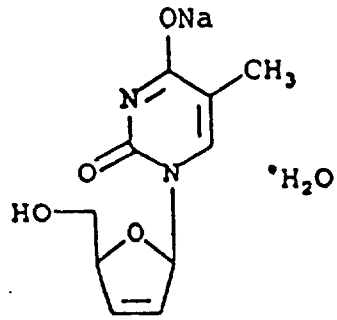

exhibiting an X-ray spectrum as shown in Figure 8.

**2.** Crystalline sodium 2',3'-dideoxyinosine monohydrate of the formula

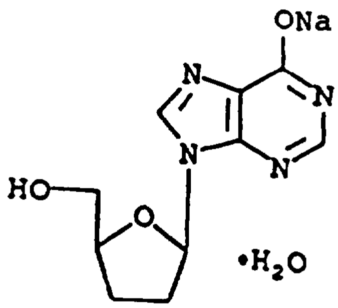

exhibiting the following X-ray d-lines:

| d | I/Io |
|---|---|
| 8.75 | 60 |
| 7.82 | 80 |
| 6.21 | 30 |
| 5.81 | 20 |
| 5.28 | 10 |
| 5.00 | 10 |
| 4.65 | 40 |
| 4.22 | 50 |
| 3.89 | 100 |
| 3.49 | 20 |
| 3.25 | 20 |
| 3.09 | 10 |
| 2.92 | 50 |
| 2.77 | 10 |
| 2.61 | 20 |

**3.** Crystalline sodium 2',3'-dideoxy-2'-fluoroinosine hemihydrate of the formula

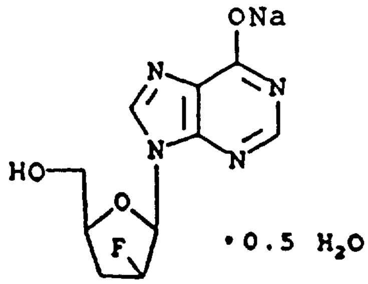

exhibiting the following X-ray d-lines:

| d | I/Io |
|---|---|
| 16.20 | 50 |
| 9.40 | 50 |
| 6.91 | 50 |
| 5.81 | 50 |
| 5.40 | 100 |

**4.** A process for preparing a pharmaceutical composition containing an antiviral effective amount of at least one salt as defined in claims 1 to 3 and a pharmaceutically acceptable carrier, which comprises providing an antiviral effective amount of at least one of said salts in a pharmaceutically acceptable carrier in a form suitable for administration.

**5.** A process according to claim 4 for preparing the composition in the form of a tablet, capsule or powder for oral administration enterically coated and buffered against gastric acid.

**6.** The use of at least one salt according to anyone of claims 1 to 3 for preparing a pharmaceutical composition for treating a virus infection.

**7.** A process for the preparation of the crystalline salts according to claims 1 to 3, which comprises reacting 2',3'-dideoxy-2',3'-didehydrothymidine, 2',3'-dideoxyinosine or 2',3'-dideoxy-2'-fluoroinosine in an inert aqueous solvent system with an appropriate base providing the sodium cation and allowing the desired salt to crystallize from the solution.

**Patentansprüche**

**Patentansprüche für folgende Vertragsstaaten : AT, BE CH, LI, DE, DK, FR, GB, IT, LU, NL, SE**

**1.** Kristallines Natrium-2',3'-didesoxy-2',3'-didehydrothymidinmonohydrat der Formel

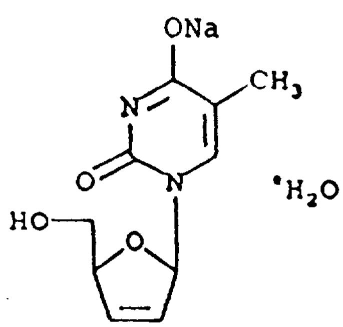

welches das in Figur 8 dargestellte Röntgenspektrum aufweist.

**2.** Kristallines Natrium-2',3'-didesoxyinosinmonohydrat der Formel

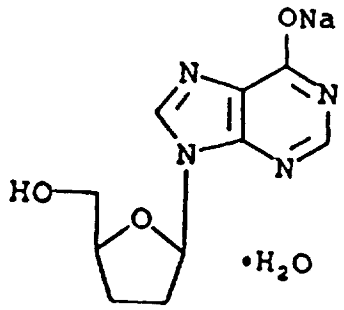

welches folgende Röntgen-d-Linien aufweist:

| d | I/Io |
|---|---|
| 8,75 | 60 |
| 7,82 | 80 |
| 6,21 | 30 |
| 5,81 | 20 |
| 5,28 | 10 |
| 5,00 | 10 |
| 4,65 | 40 |

| d | I/Io |
|---|---|
| 4,22 | 50 |
| 3,89 | 100 |
| 3,49 | 20 |
| 3,25 | 20 |
| 3,09 | 10 |
| 2,92 | 50 |
| 2,77 | 10 |
| 2,61 | 20 |

**3.** Kristallines Natrium-2',3'-didesoxy-2'-fluorinosinhemihydrat der Formel

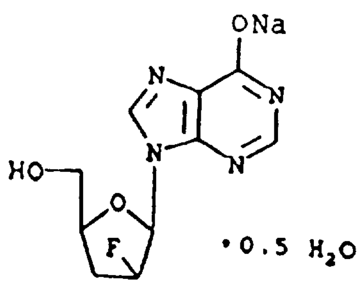

welches folgende Röntgen-d-Linien aufweist:

| d | I/Io |
|---|---|
| 16,20 | 50 |
| 9,40 | 50 |
| 6,91 | 50 |
| 5,81 | 50 |
| 5,40 | 100 |

**4.** Pharmazeutisches Mittel, enthaltend eine antiviral wirksame Menge wenigstens eines Salzes nach einem der Ansprüche 1 bis 3 und einen pharmazeutisch verträglichen Träger.

**5.** Mittel nach Anspruch 4 in Tabletten-, Kapsel- oder Pulverform zur oralen Verabreichung, enterisch beschichtet und gegen Magensäure gepuffert.

**6.** Verfahren zur Herstellung des Mittels nach Anspruch 4 oder 5, umfassend die Bereitstellung einer antiviral wirksamen Menge wenigstens eines Salzes nach einem der Ansprüche 1 bis 3 in einem pharmazeutisch verträglichen Träger in einer zur Verabreichung geeigneten Form, vorzugsweise in Form einer Tablette, einer Kapsel oder eines Pulvers zur oralen Verabreichung, enterisch beschichtet und gegen Magensäure gepuffert.

**7.** Verwendung wenigstens eines Salzes nach einem der Ansprüche 1 bis 3 zur Herstellung eines pharmazeutischen Mittels zur Behandlung einer Virusinfektion.

**8.** Verfahren zur Herstellung der kristallinen Salze der Ansprüche 1 bis 3, umfassend die Umsetzung von 2',3'-Didesoxy-2',3'-didehydrothymidin, 2',3'-Didesoxyinosin oder 2',3'-Didesoxy-2'-fluorinosin in einem inerten wäßrigen Lösungsmittelsystem mit einer geeigneten Base, die das Natriumkation liefert und die Kristallisation des gewünschten Salzes aus der Lösung ermöglicht.

**Patentansprüche für folgenden Vertragsstaat : ES**

**1.** Verfahren zur Herstellung eines kristallinen Natrium-2',3'-didesoxy-2',3'-didehydrothymidinmonohydrates der Formel

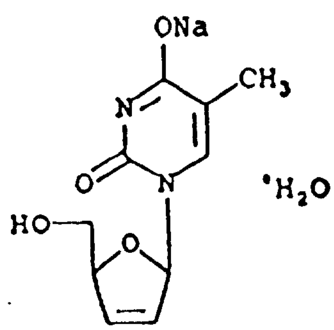

welches das in Figur 8 dargestellte Röntgenspektrum aufweist,
wobei 2',3'-Didesoxy-2',3'-didehydrothymidin, in einem inerten wäßrigen Lösungsmittelsystem mit einer geeigneten Base umgesetzt wird, die das Natriumkation liefert und die Kristallisation des gewünschten Salzes aus der Lösung ermöglicht.

**2.** Verfahren zur Herstellung eines kristallinen Natrium-2',3'-didesoxyinosinmonohydrates der Formel

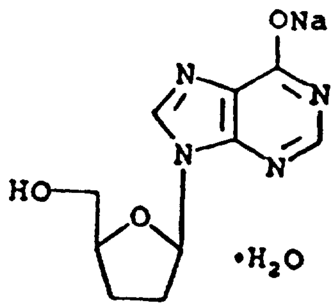

welches folgende Röntgen-d-Linien aufweist:

| d | I/Io |
|---|---|
| 8,75 | 60 |
| 7,82 | 80 |
| 6,21 | 30 |
| 5,81 | 20 |
| 5,28 | 10 |
| 5,00 | 10 |
| 4,65 | 40 |
| 4,22 | 50 |
| 3,89 | 100 |
| 3,49 | 20 |
| 3,25 | 20 |
| 3,09 | 10 |
| 2,92 | 50 |
| 2,77 | 10 |
| 2,61 | 20, |

wobei 2',3'-Didesoxyinosin in einem inerten wäßrigen Lösungsmittelsystem mit einer geeigneten Base umgesetzt wird, die das Natriumkation liefert und die Kristallisation des gewünschten Salzes aus der Lösung ermöglicht.

**3.** Verfahren zur Herstellung eines kristallinen Natrium-2',3'-didesoxy-2'-fluorinosinhemihydrates der Formel

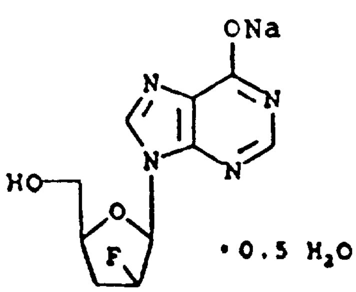

welches folgende Röntgen-d-Linien aufweist:

| d | I/Io |
|---|---|
| 16,20 | 50 |
| 9,40 | 50 |
| 6,91 | 50 |

(fortgesetzt)

| d | I/Io |
|---|---|
| 5,81 | 50 |
| 5,40 | 100, |

wobei 2',3'-Didesoxy-2'-fluorinosin in einem inerten wäßrigen Lösungsmittelsystem mit einer geeigneten Base umgesetzt wird, die das Natriumkation liefert und die Kristallisation des gewünschten Salzes aus der Lösung ermöglicht.

**4.** Verfahren zur Herstellung eines pharmazeutischen Mittels, enthaltend eine antiviral wirksame Menge wenigstens eines Salzes nach einem der Ansprüche 1 bis 3 und einen pharmazeutisch verträglichen Träger, wobei eine antiviral wirksame Menge wenigstens eines dieser Salze in einem pharmazeutisch verträglichen Träger in einer zur Verabreichung geeigneten Form bereitgestellt wird.

**5.** Verfahren nach Anspruch 4 zur Herstellung des Mittels in Tabletten-, Kapsel- oder Pulverform zur oralen Verabreichung, enterisch beschichtet und gegen Magensäure gepuffert.

**6.** Verwendung wenigstens eines Salzes nach einem der Ansprüche 1 bis 3 zur Herstellung eines pharmazeutischen Mittels zur Behandlung einer Virusinfektion.

**Patentansprüche für folgenden Vertragsstaat : GR**

**1.** Kristallines Natrium-2',3'-didesoxy-2',3'-didehydrothymidinmonohydrat der Formel

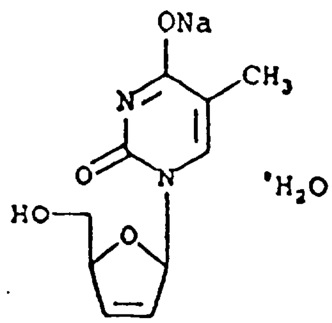

welches das in Figur 8 dargestellte Röntgenspektrum aufweist.

**2.** Kristallines Natrium-2',3'-didesoxyinosinmonohydrat der Formel

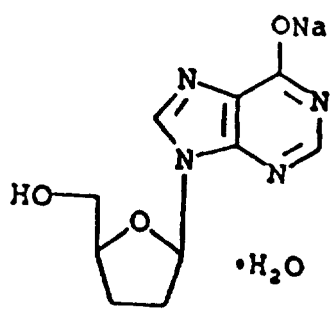

welches folgende Röntgen-d-Linien aufweist:

| d | I/Io |
|---|---|
| 8,75 | 60 |
| 7,82 | 80 |
| 6,21 | 30 |
| 5,81 | 20 |
| 5,28 | 10 |
| 5,00 | 10 |
| 4,65 | 40 |

| d | I/Io |
|---|---|
| 4,22 | 50 |
| 3,89 | 100 |
| 3,49 | 20 |
| 3,25 | 20 |
| 3,09 | 10 |
| 2,92 | 50 |
| 2,77 | 10 |
| 2,61 | 20 |

**3.** Kristallines Natrium-2',3'-didesoxy-2'-fluorinosinhemihydrat der Formel

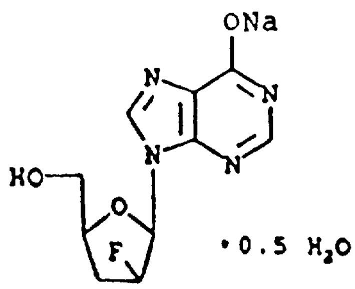

welches folgende Röntgen-d-Linien aufweist:

| d | I/Io |
|---|---|
| 16,20 | 50 |
| 9,40 | 50 |
| 6,91 | 50 |
| 5,81 | 50 |
| 5,40 | 100 |

**4.** Verfahren zur Herstellung eines pharmazeutischen Mittels, enthaltend eine antiviral wirksame Menge wenigstens eines Salzes nach einem der Ansprüche 1 bis 3 und einen pharmazeutisch verträglichen Träger, wobei eine antiviral wirksame Menge wenigstens eines dieser Salze in einem pharmazeutisch verträglichen Träger in einer zur Verabreichung geeigneten Form bereitgestellt wird.

**5.** Verfahren nach Anspruch 4 zur Herstellung des Mittels in Tabletten-, Kapsel- oder Pulverform zur oralen Verabreichung, enterisch beschichtet und gegen Magensäure gepuffert.

**6.** Verwendung wenigstens eines Salzes nach einem der Ansprüche 1 bis 3 zur Herstellung eines pharmazeutischen Mittels zur Behandlung einer Virusinfektion.

7. Verfahren zur Herstellung der kristallinen Salze der Ansprüche 1 bis 3, umfassend die Umsetzung von 2',3'-Didesoxy-2',3'-didehydrothymidin, 2',3'-Didesoxyinosin oder 2',3'-Didesoxy-2'-fluorinosin in einem inerten wäßrigen Lösungsmittelsystem mit einer geeigneten Base, die das Natriumkation liefert und die Kristallisation des gewünschten Salzes aus der Lösung ermöglicht.

## Revendications

**Revendications pour les Etats contractants suivants : AT, BE, CH, LI, DE, DK, FR, GB, IT, LU, NL, SE**

1. Sodium 2',3'-didéoxy-2',3'-didéhydrothymidine monohydrate cristallin de formule

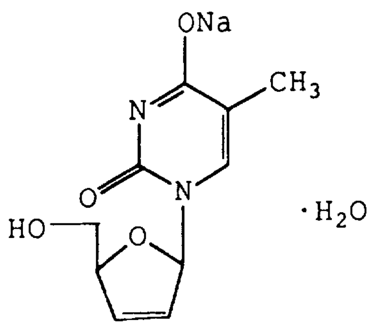

présentant un spectre des rayons-X tel que montré en Figure 8.

2. Sodium 2',3'-didéoxyinosine monohydrate cristallin de formule

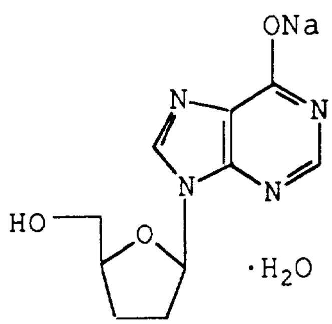

présentant les lignes d de rayons-X suivantes :

| d | I/Io |
|---|---|
| 8,75 | 60 |
| 7,82 | 80 |
| 6,21 | 30 |
| 5,81 | 20 |
| 5,28 | 10 |
| 5,00 | 10 |
| 4,65 | 40 |
| 4,22 | 50 |
| 3,89 | 100 |
| 3,49 | 20 |
| 3,25 | 20 |

| d | I/Io |
|---|---|
| 3,09 | 10 |
| 2,92 | 50 |
| 2,77 | 10 |
| 2,61 | 20 |

**3.** Sodium 2',3'-didéoxy-2'-fluoroinosine hémihydrate cristallin de formule

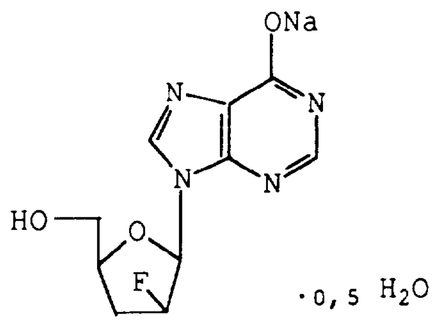

présentant les lignes d des rayons-X suivantes :

| d | I/Io |
|---|---|
| 16,20 | 50 |
| 9,40 | 50 |
| 6,91 | 50 |
| 5,81 | 50 |
| 5,40 | 100 |

**4.** Composition pharmaceutique contenant une quantité efficace antivirale d'au moins un sel selon l'une quelconque des revendications 1 à 3 et un porteur pharmaceutiquement acceptable.

**5.** Composition selon la revendication 4 sous la forme d'un comprimé, d'une capsule ou d'une poudre pour une administration orale entériquement revêtu et tamponné contre l'acide gastrique.

**6.** Procédé pour préparer la composition selon la revendication 4 ou 5 qui comprend la fourniture d'une quantité efficace antivirale d'au moins un sel selon l'une quelconque des revendications 1 à 3 dans un porteur pharmaceutiquement acceptable sous une forme appropriée pour une administration, de préférence sous la forme d'un comprimé, d'une capsule ou d'une poudre pour une administration orale entériquement revêtu et tamponné contre l'acide gastrique.

**7.** Utilisation d'au moins un sel selon selon l'une quelconque des revendications 1 à 3 pour préparer une composition pharmaceutique pour traiter une infection par un virus.

**8.** Procédé pour la préparation des sels cristallins selon les revendications 1 à 3, qui comprend la réaction de la 2',3'-didéoxy-2',3'-didéhydrothymidine, de la 2',3'-didéoxyinosine ou de la 2',3'-didéoxy-2'-fluoroinosine dans un système de solvant aqueux inerte avec une base appropriée fournissant le cation sodium et le fait de laisser le sel désiré cristalliser à partir de la solution.

**Revendications pour l'Etat contractant suivant : ES**

**1.** Procédé pour la préparation de sodium 2',3'-didéoxy-2',3'-didéhydrothymidine monohydrate cristallin de formule

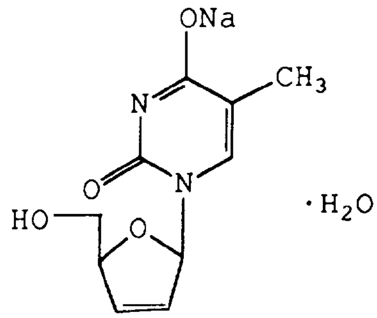

présentant un spectre des rayons-X tel que montré en Figure 8, qui comprend la réaction de 2',3'-didéoxy-2',3'-didéhydrothymidine dans un système de solvant aqueux inerte avec une base appropriée fournissant le cation sodium et le fait de laisser le sel désiré cristalliser à partir de la solution.

**2.** Procédé pour la préparation de sodium 2',3'-didéoxyinosine monohydrate cristallin de formule

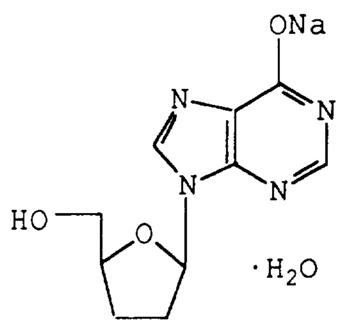

présentant les lignes d de rayons-X suivantes:

| d | I/Io |
|---|---|
| 8,75 | 60 |
| 7,82 | 80 |
| 6,21 | 30 |
| 5,81 | 20 |
| 5,28 | 10 |
| 5,00 | 10 |
| 4,65 | 40 |
| 4,22 | 50 |
| 3,89 | 100 |
| 3,49 | 20 |
| 3,25 | 20 |
| 3,09 | 10 |
| 2,92 | 50 |
| 2,77 | 10 |
| 2,61 | 20 |

qui comprend la réaction de 2',3'-didéoxyinosine dans un système de solvant aqueux inerte avec une base appropriée fournissant le cation sodium et le fait de laisser le sel désiré cristalliser à partir de la solution.

**3.** Procédé pour la préparation de sodium 2',3'-didéoxy-2'-fluoroinosine hémihydrate cristallin de formule

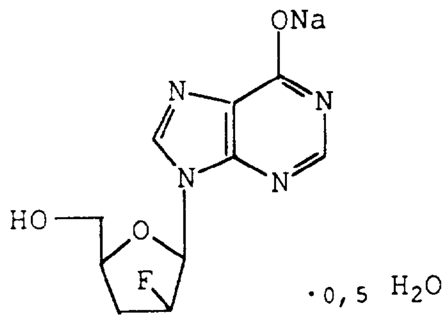

présentant les lignes d de rayons X suivantes:

| d | I/Io |
|---|---|
| 16,20 | 50 |
| 9,40 | 50 |
| 6,91 | 50 |
| 5,81 | 50 |
| 5,40 | 100 |

qui comprend la réaction de 2',3'-didéoxy-2'-fluoroinosine dans un système de solvant aqueux inerte avec une base appropriée fournissant le cation sodium et le fait de laisser le sel désiré cristalliser à partir de la solution.

**4.** Procédé pour préparer une composition pharmaceutique contenant une quantité efficace antivirale d'au moins un sel tel que défini dans l'une quelconque des revendications 1 à 3 et un porteur pharmaceutiquement acceptable, qui comprend la fourniture d'une quantité efficace antivirale d'au moins un desdits sels dans un porteur pharmaceutiquement acceptable sous une forme appropriée pour une administration.

**5.** Procédé selon la revendication 4 pour préparer la composition sous la forme d'un comprimé, d'une capsule ou d'une poudre pour une administration orale entériquement revêtu et tamponné contre l'acide gastrique.

**6.** Utilisation d'au moins un sel selon l'une quelconque des revendications 1 à 3 pour préparer une composition pharmaceutique pour traiter une infection par un virus.

**Revendications pour l'Etat contractant suivant : GR**

**1.** Sodium 2',3'-didéoxy-2',3'-didéhydrothymidine monohydrate cristallin de formule

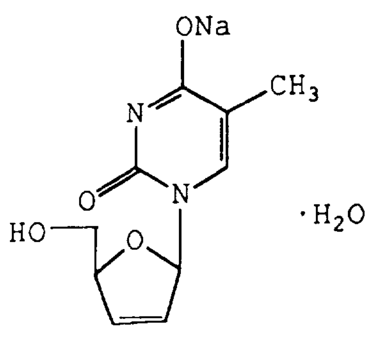

présentant un spectre des rayons-X tel que montré en Figure 8.

**2.** Sodium 2',3'-didéoxyinosine monohydrate critallin de formule

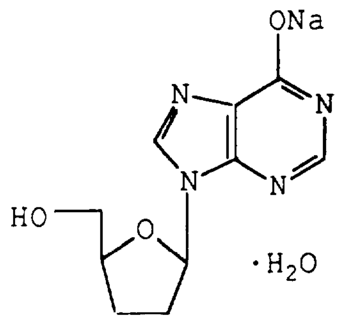

présentant les lignes d des rayons-X suivantes :

| d | I/Io |
|---|---|
| 8,75 | 60 |
| 7,82 | 80 |
| 6,21 | 30 |
| 5,81 | 20 |
| 5,28 | 10 |
| 5,00 | 10 |
| 4,65 | 40 |
| 4,22 | 50 |

| d | I/Io |
|---|---|
| 3,89 | 100 |
| 3,49 | 20 |
| 3,25 | 20 |
| 3,09 | 10 |
| 2,92 | 50 |
| 2,77 | 10 |
| 2,61 | 20 |

**3.** Sodium 2',3'-didéoxy-2'-fluoroinosine hémihydrate cristallin de formule

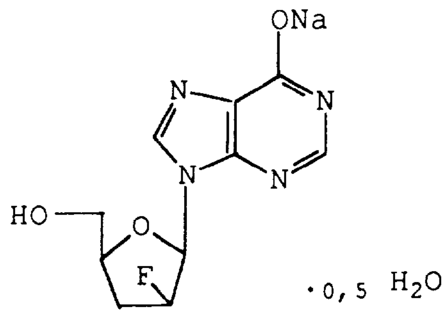

présentant les lignes d de rayons-X suivantes :

| d | I/Io |
|---|---|
| 16,20 | 50 |
| 9,40 | 50 |
| 6,91 | 50 |

(suite)

| d | I/Io |
|---|---|
| 5,81 | 50 |
| 5,40 | 100 |

**4.** Procédé pour préparer une composition pharmaceutique contenant une quantité antivirale efficace d'au moins un sel tel que défini dans les revendications 1 à 3 et un porteur pharmaceutiquement acceptable, qui comprend la fourniture d'une quantité antivirale efficace d'au moins un desdits sels dans un porteur pharmaceutiquement acceptable sous une forme appropriée pour une administration.

**5.** Procédé selon la revendication 4 pour préparer la composition sous la forme d'un comprimé, d'une capsule ou d'une poudre pour une administration orale entériquement revêtu et tamponné contre l'acide gastrique.

**6.** Utilisation d'au moins un sel selon l'une quelconque des revendications 1 à 3 pour préparer une composition pharmaceutique pour traiter une infection par un virus.

**7.** Procédé pour la préparation des sels cristallins selon les revendications 1 à 3, qui comprend la réaction de 2',3'-didéoxy-2',3'-didéhydrothymidine, de 2',3'-didéoxyinosine ou de 2',3'-didéoxy-2'-fluoroinosine dans un système de solvant aqueux inerte avec une base appropriée fournissant le cation sodium et le fait de laisser le sel désiré cristalliser à partir de la solution.

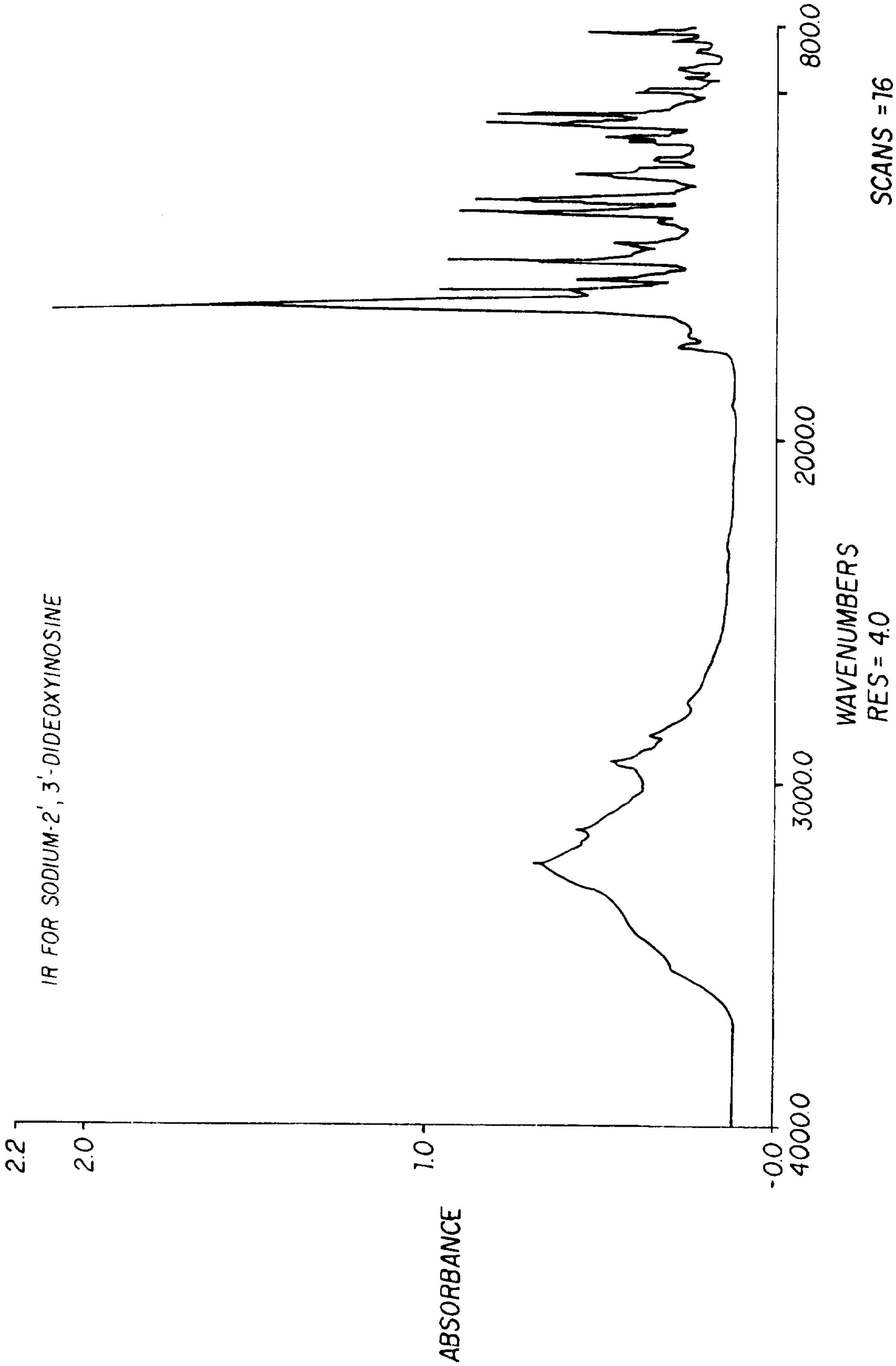
IR FOR SODIUM-2', 3'-DIDEOXYINOSINE
ABSORBANCE
2.2
2.0
1.0
-0.0
4000.0
3000.0
2000.0
800.0
WAVENUMBERS
RES = 4.0
SCANS =16

FIG. 1

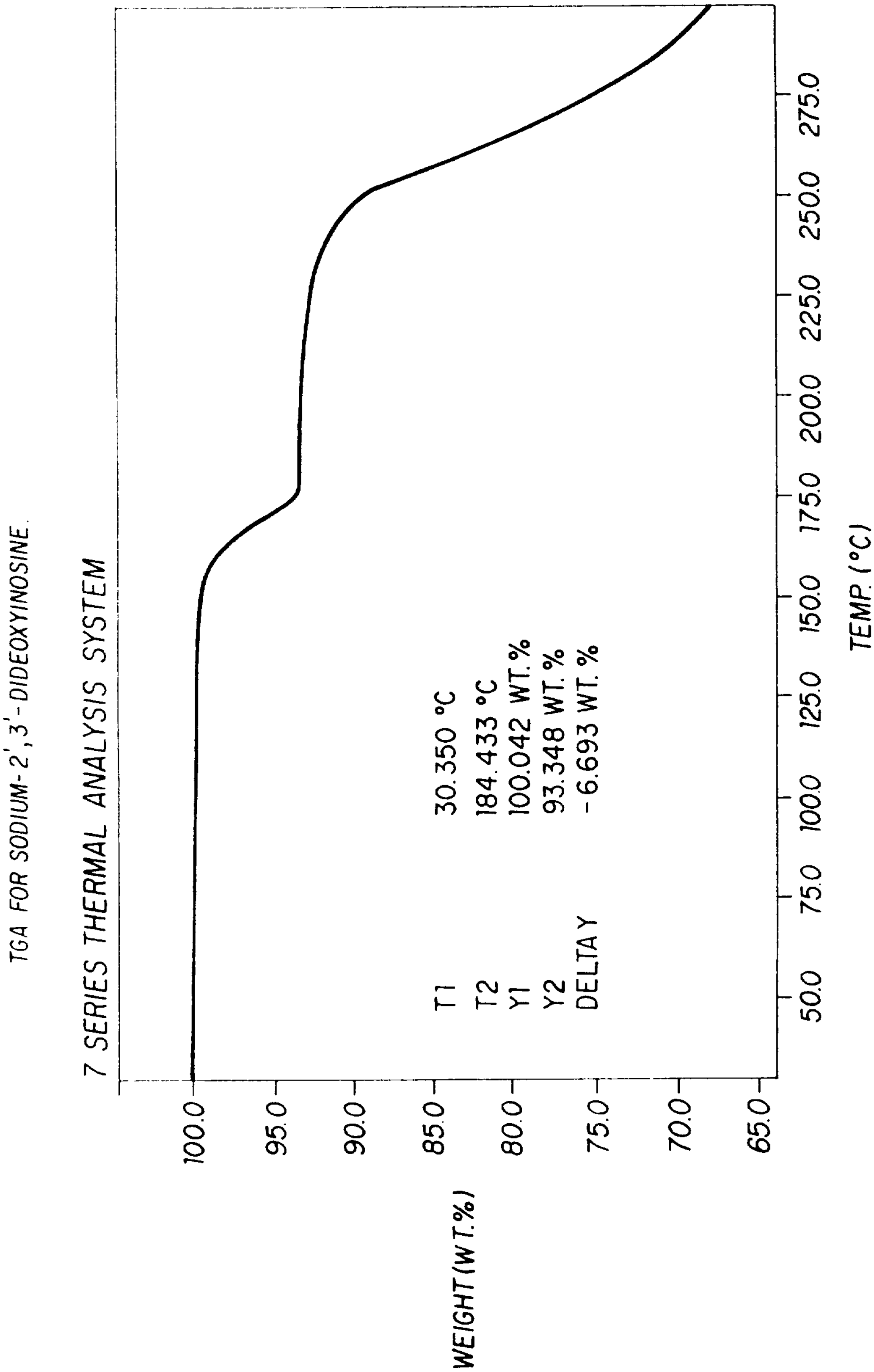
TGA FOR SODIUM-2',3'-DIDEOXYINOSINE.
7 SERIES THERMAL ANALYSIS SYSTEM
T1 30.350 °C
T2 184.433 °C
Y1 100.042 WT. %
Y2 93.348 WT. %
DELTA Y -6.693 WT. %
WEIGHT (WT.%)
100.0
95.0
90.0
85.0
80.0
75.0
70.0
65.0
50.0
75.0
100.0
125.0
150.0
175.0
200.0
225.0
250.0
275.0
TEMP. (°C)

FIG. 2

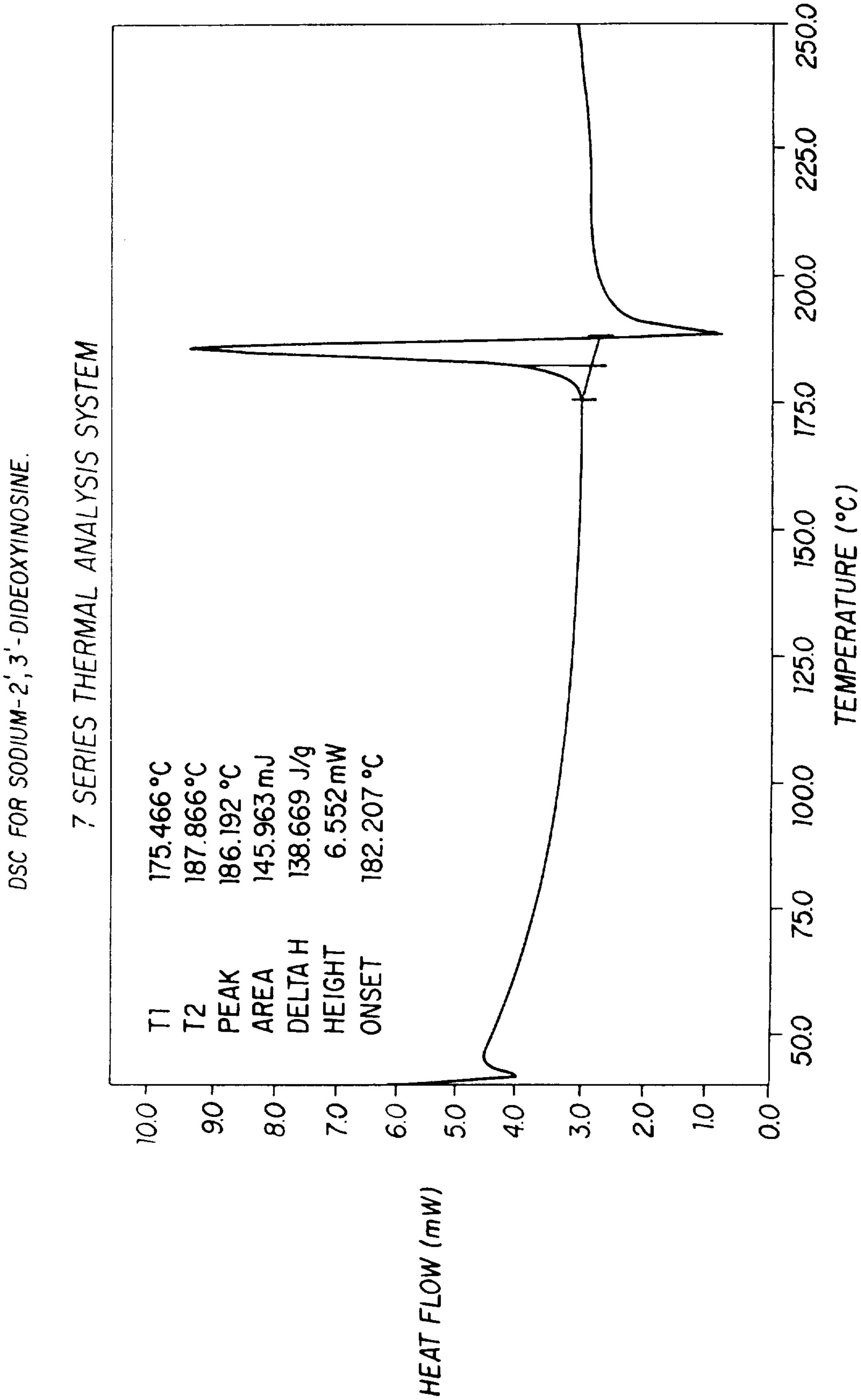
DSC FOR SODIUM-2',3'-DIDEOXYINOSINE.
7 SERIES THERMAL ANALYSIS SYSTEM
T1 175.466 °C
T2 187.866 °C
PEAK 186.192 °C
AREA 145.963 mJ
DELTA H 138.669 J/g
HEIGHT 6.552 mW
ONSET 182.207 °C
HEAT FLOW (mW)
TEMPERATURE (°C)
10.0
9.0
8.0
7.0
6.0
5.0
4.0
3.0
2.0
1.0
0.0
50.0
75.0
100.0
125.0
150.0
175.0
200.0
225.0
250.0

FIG. 3

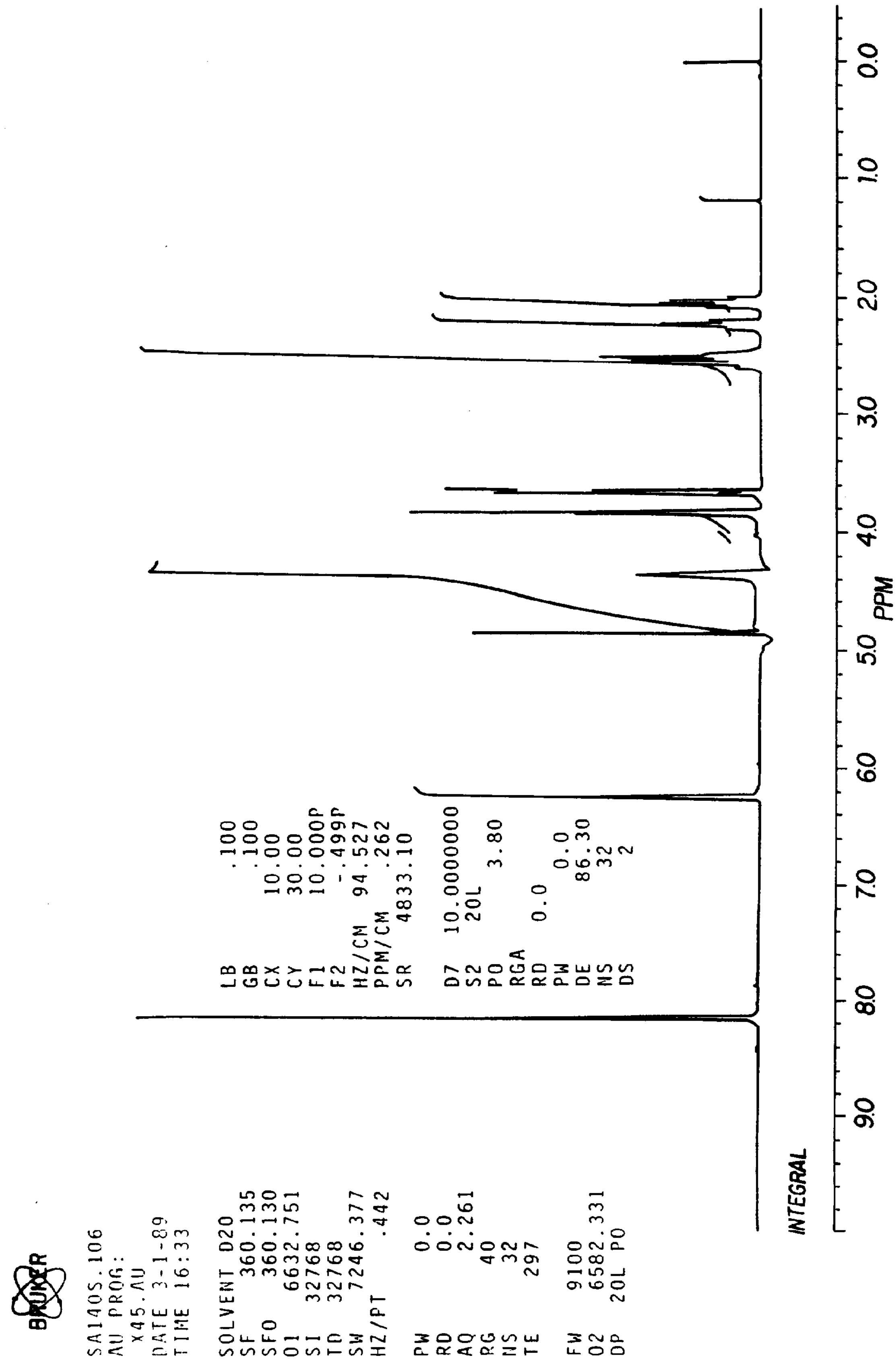
BRUKER
SA140S.106
AU PROG:
X45.AU
DATE 3-1-89
TIME 16:33
SOLVENT D2O
SF 360.135
SFO 360.130
O1 6632.751
SI 32768
TD 32768
SW 7246.377
HZ/PT .442
PW 0.0
RD 0.0
AQ 2.261
RG 40
NS 32
TE 297
FW 9100
O2 6582.331
DP 20L PO
LB .100
GB .100
CX 10.00
CY 30.00
F1 10.000P
F2 -.499P
HZ/CM 94.527
PPM/CM .262
SR 4833.10
D7 10.0000000
S2 20L
PO 3.80
RGA
RD 0.0
PW 0.0
DE 86.30
NS 32
DS 2
INTEGRAL
9.0
8.0
7.0
6.0
5.0
4.0
3.0
2.0
1.0
0.0
PPM

FIG. 4

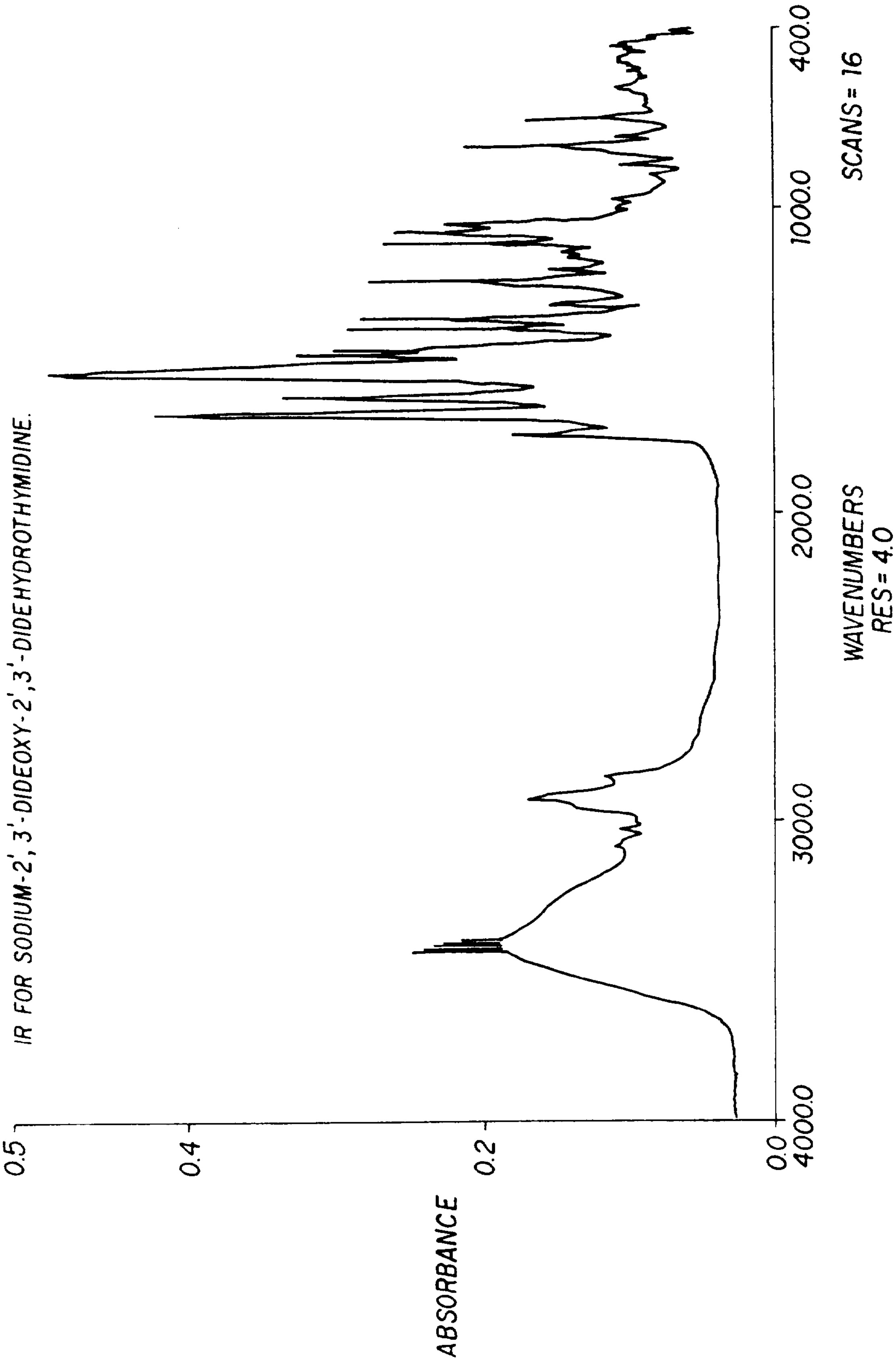
IR FOR SODIUM-2',3'-DIDEOXY-2',3'-DIDEHYDROTHYMIDINE.
ABSORBANCE
0.5
0.4
0.2
0.0
4000.0
3000.0
2000.0
1000.0
400.0
WAVENUMBERS
RES = 4.0
SCANS = 16

FIG. 5

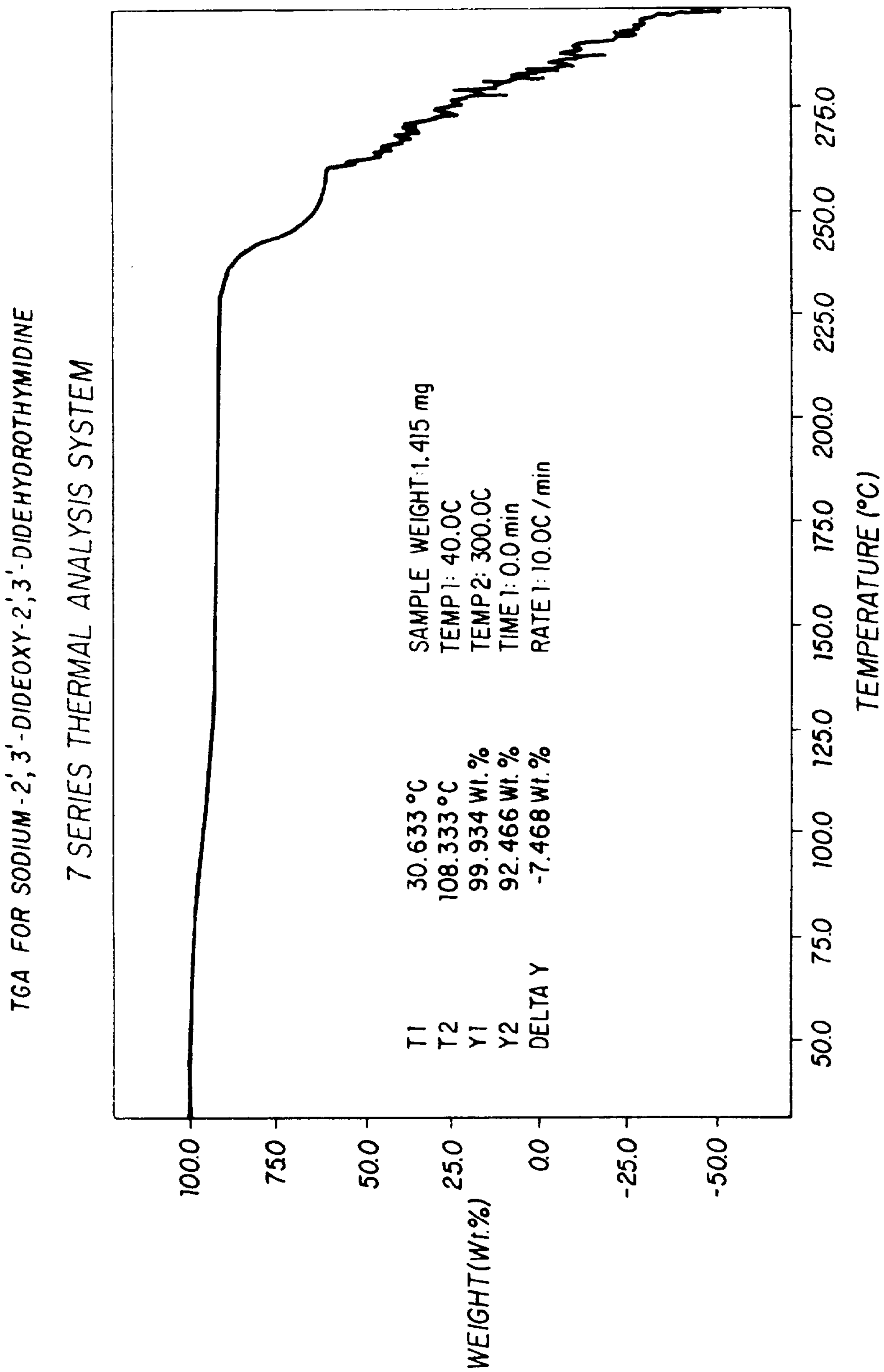
TGA FOR SODIUM-2',3'-DIDEOXY-2',3'-DIDEHYDROTHYMIDINE
7 SERIES THERMAL ANALYSIS SYSTEM
T1 30.633 °C
T2 108.333 °C
Y1 99.934 Wt.%
Y2 92.466 Wt.%
DELTA Y -7.468 Wt.%
SAMPLE WEIGHT: 1.415 mg
TEMP 1: 40.0C
TEMP 2: 300.0C
TIME 1: 0.0 min
RATE 1: 10.0C / min
WEIGHT (Wt.%)
100.0
75.0
50.0
25.0
0.0
-25.0
-50.0
TEMPERATURE (°C)
50.0
75.0
100.0
125.0
150.0
175.0
200.0
225.0
250.0
275.0

FIG. 6

DSC FOR SODIUM-2',3'-DIDEOXY-2',3'-DIDEHYDROTHYMIDINE.

7 SERIES THERMAL ANALYSIS SYSTEM

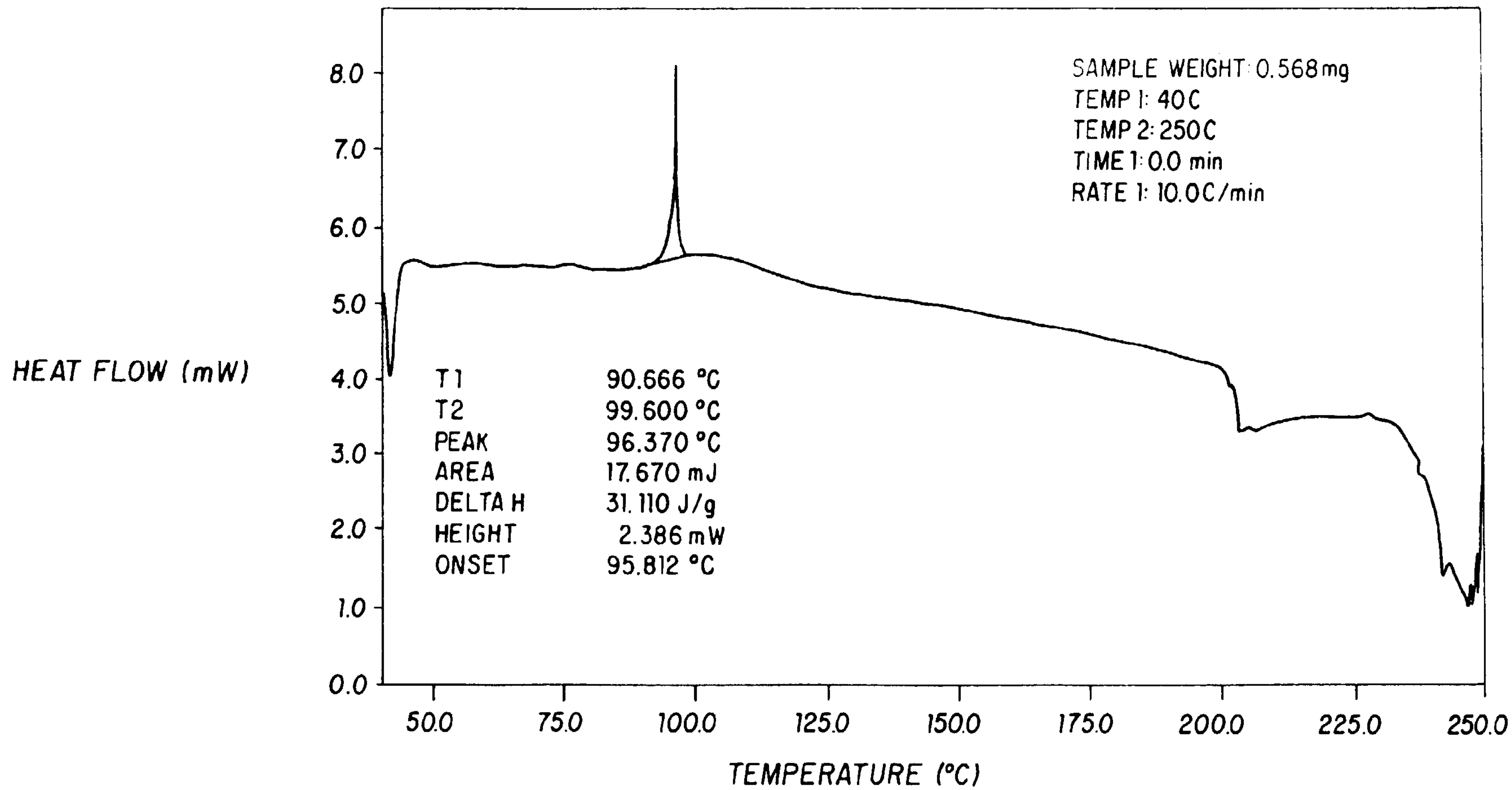


FIG. 7

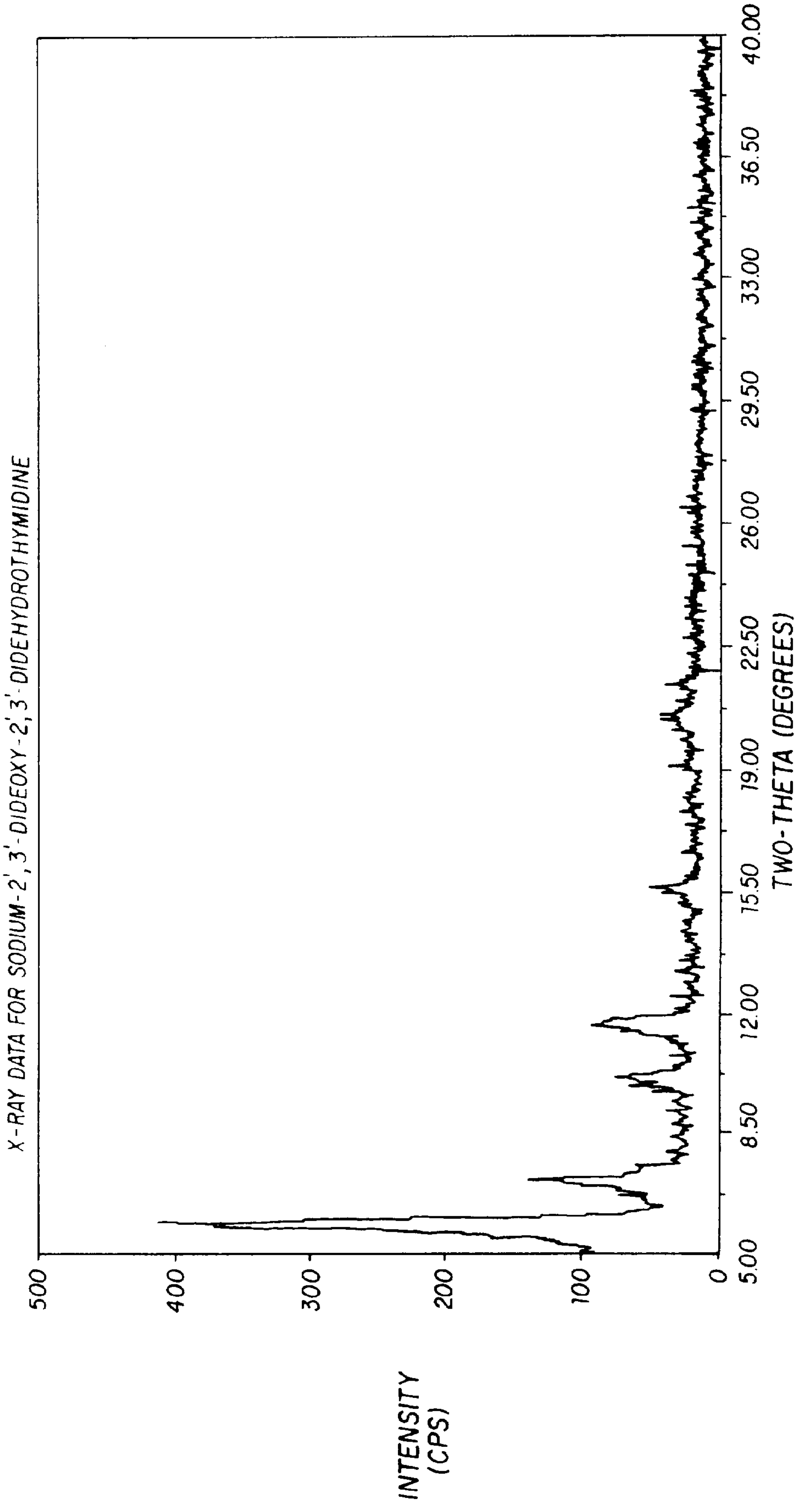
X-RAY DATA FOR SODIUM-2',3'-DIDEOXY-2',3'-DIDEHYDROTHYMIDINE
INTENSITY (CPS)
500
400
300
200
100
0
5.00
8.50
12.00
15.50
19.00
22.50
26.00
29.50
33.00
36.50
40.00
TWO-THETA (DEGREES)

FIG. 8

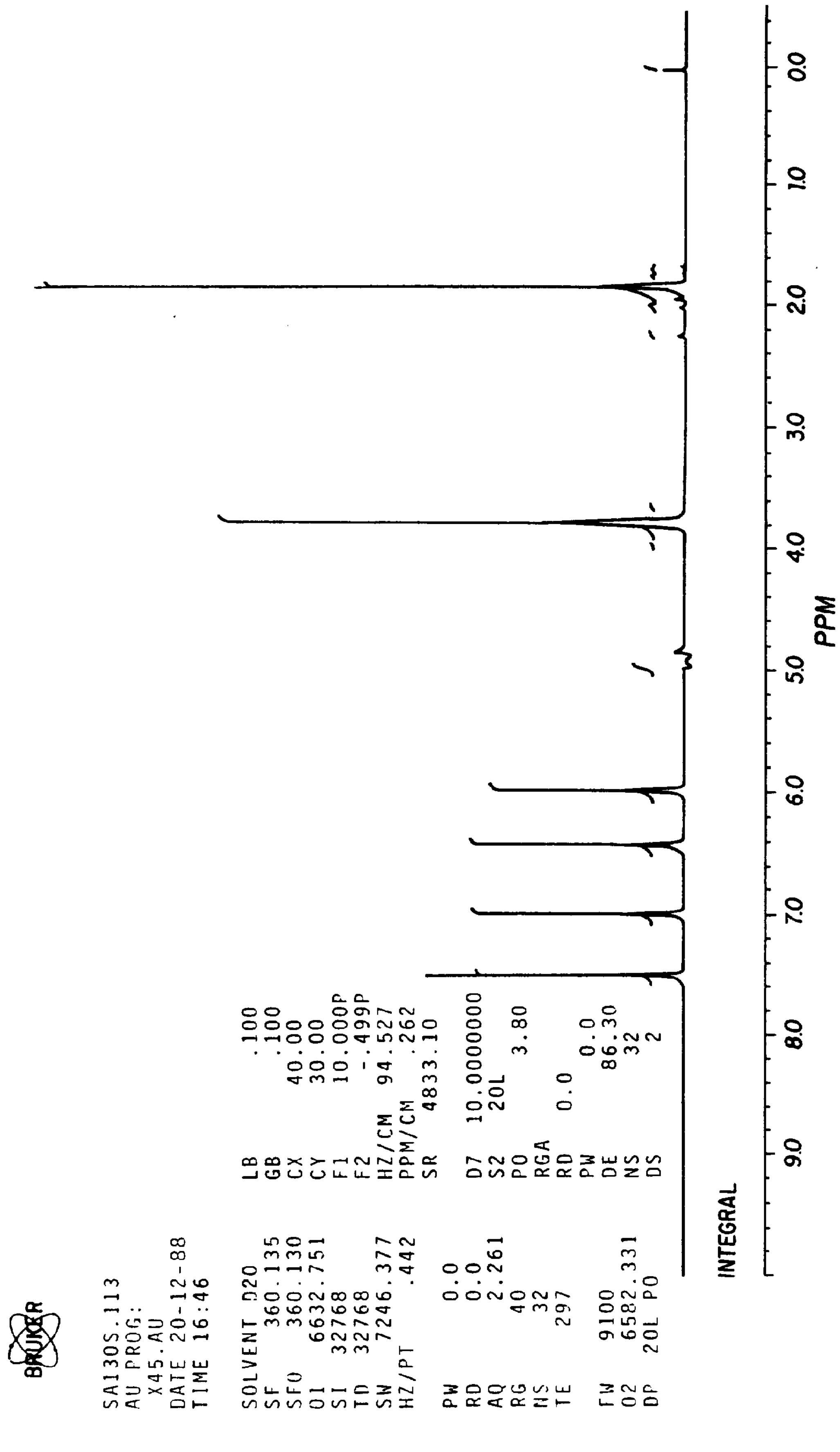
BRUKER
SA130S.113
AU PROG:
X45.AU
DATE 20-12-88
TIME 16:46
SOLVENT D20
SF 360.135
SFO 360.130
O1 6632.751
SI 32768
TD 32768
SW 7246.377
HZ/PT .442
PW 0.0
RD 0.0
AQ 2.261
RG 40
NS 32
TE 297
FW 9100
O2 6582.331
DP 20L PO
LB .100
GB .100
CX 40.00
CY 30.00
F1 10.000P
F2 -.499P
HZ/CM 94.527
PPM/CM .262
SR 4833.10
D7 10.0000000
S2 20L
PO 3.80
RGA
RD 0.0
PW 0.0
DE 86.30
NS 32
DS 2
INTEGRAL
9.0
8.0
7.0
6.0
5.0
4.0
3.0
2.0
1.0
0.0
PPM

FIG. 9

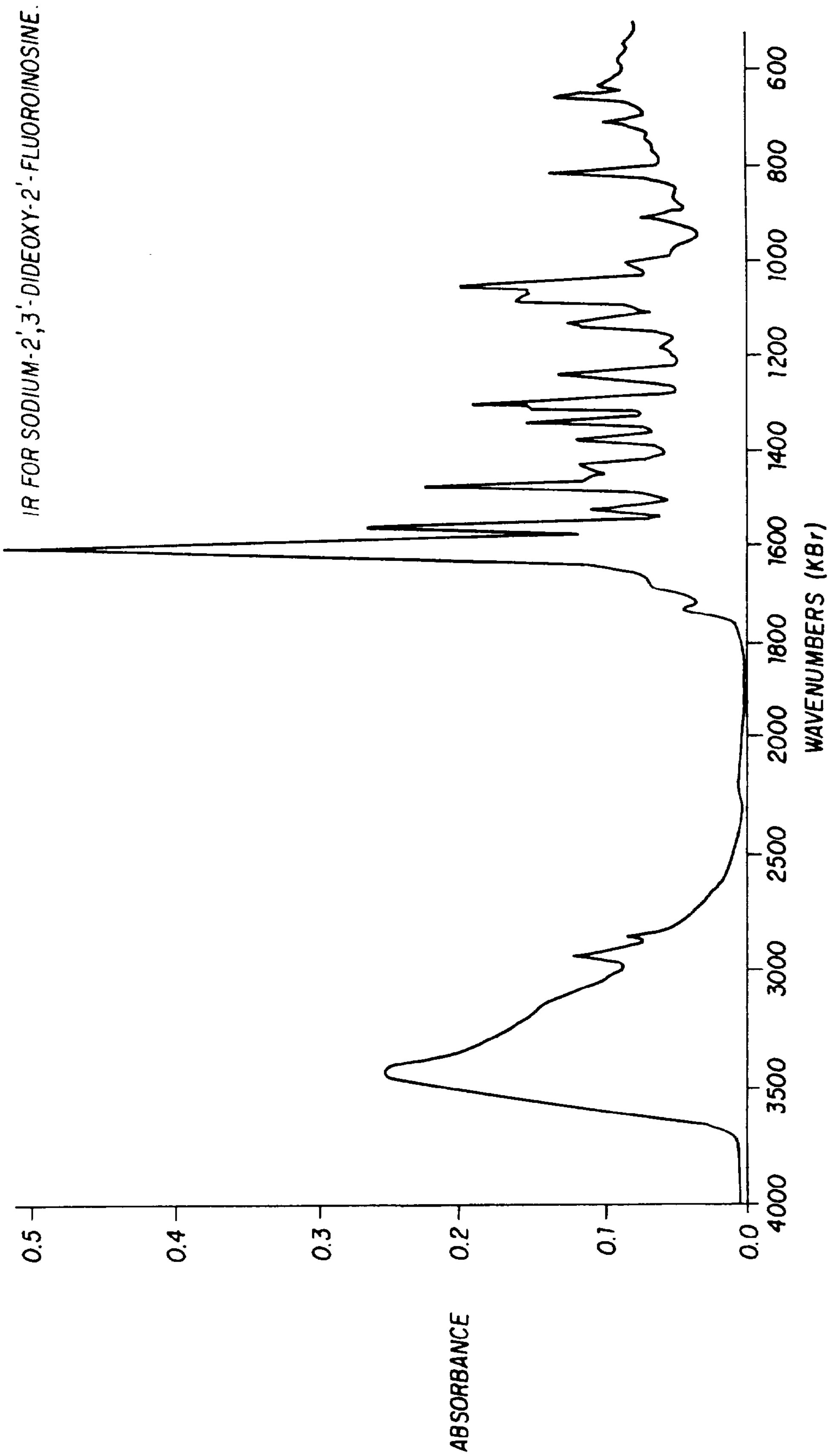
IR FOR SODIUM-2',3'-DIDEOXY-2'-FLUOROINOSINE.
ABSORBANCE
0.5
0.4
0.3
0.2
0.1
0.0
4000
3500
3000
2500
2000
1800
1600
1400
1200
1000
800
600
WAVENUMBERS (KBr)

FIG. 10

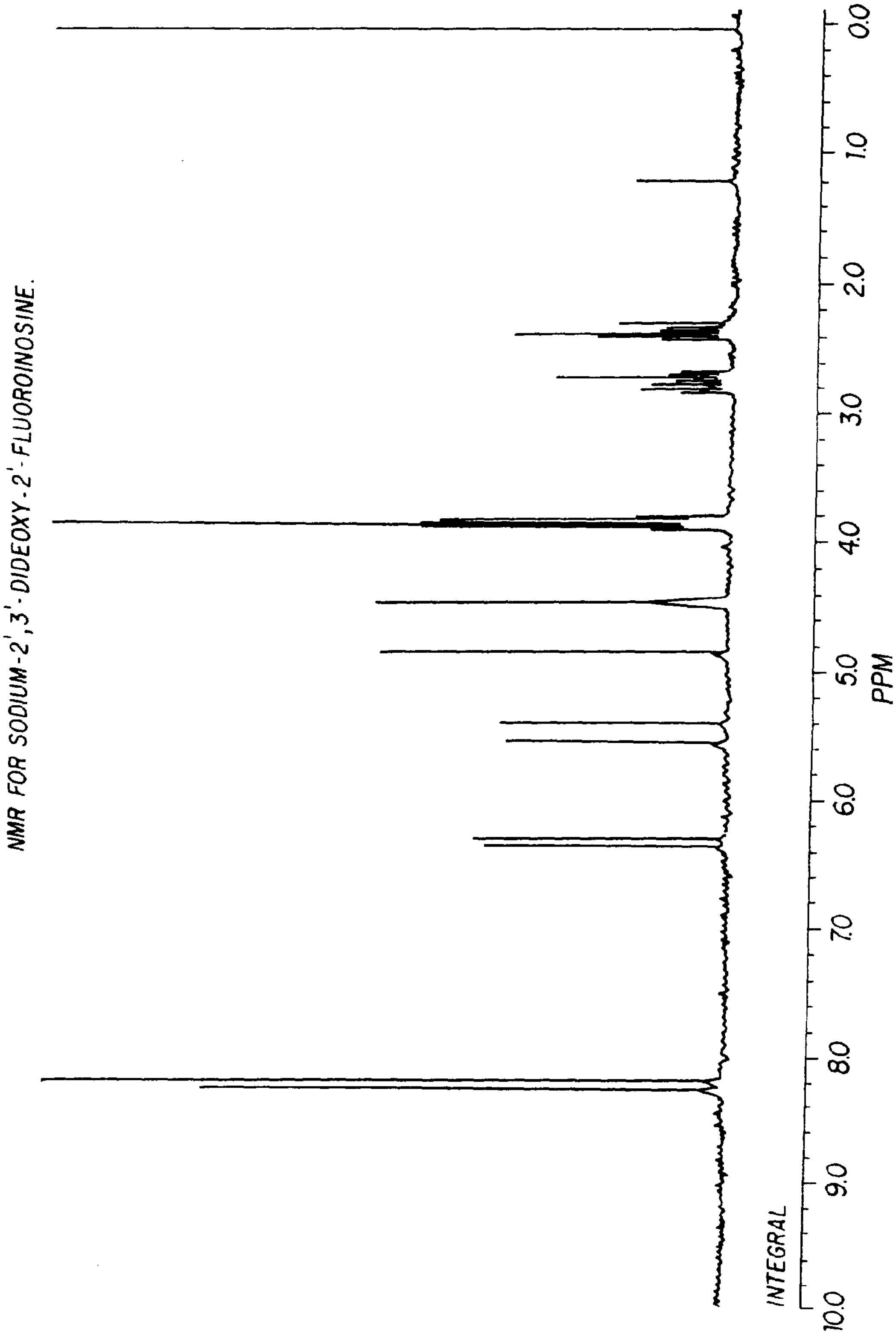
NMR FOR SODIUM-2',3'-DIDEOXY-2'-FLUOROINOSINE.
INTEGRAL
10.0
9.0
8.0
7.0
6.0
5.0
PPM
4.0
3.0
2.0
1.0
0.0

FIG. 11

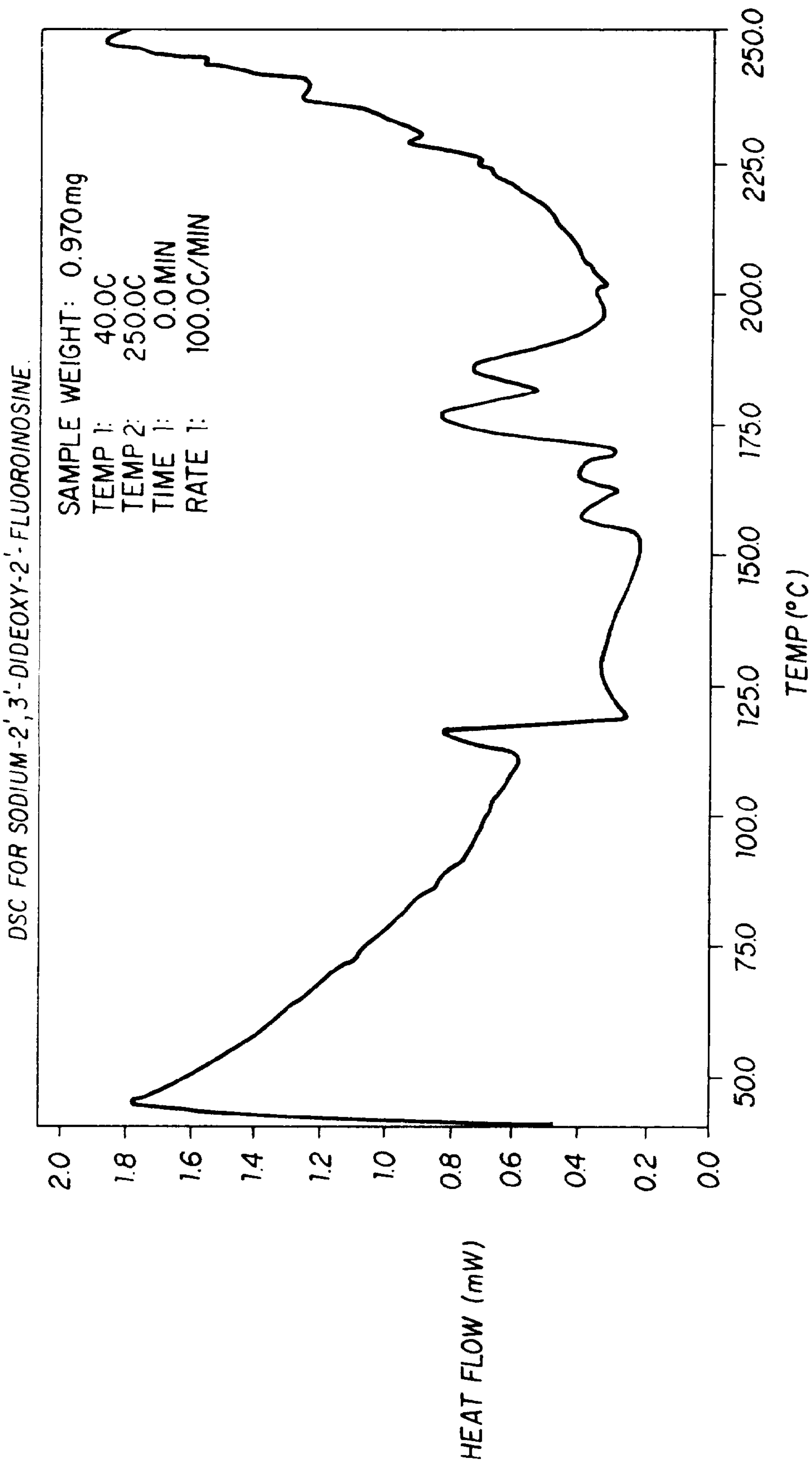
DSC FOR SODIUM-2',3'-DIDEOXY-2'-FLUOROINOSINE.
SAMPLE WEIGHT: 0.970mg
TEMP 1: 40.0C
TEMP 2: 250.0C
TIME 1: 0.0MIN
RATE 1: 100.0C/MIN
HEAT FLOW (mW)
2.0
1.8
1.6
1.4
1.2
1.0
0.8
0.6
0.4
0.2
0.0
50.0
75.0
100.0
125.0
150.0
175.0
200.0
225.0
250.0
TEMP (°C)

FIG. 12

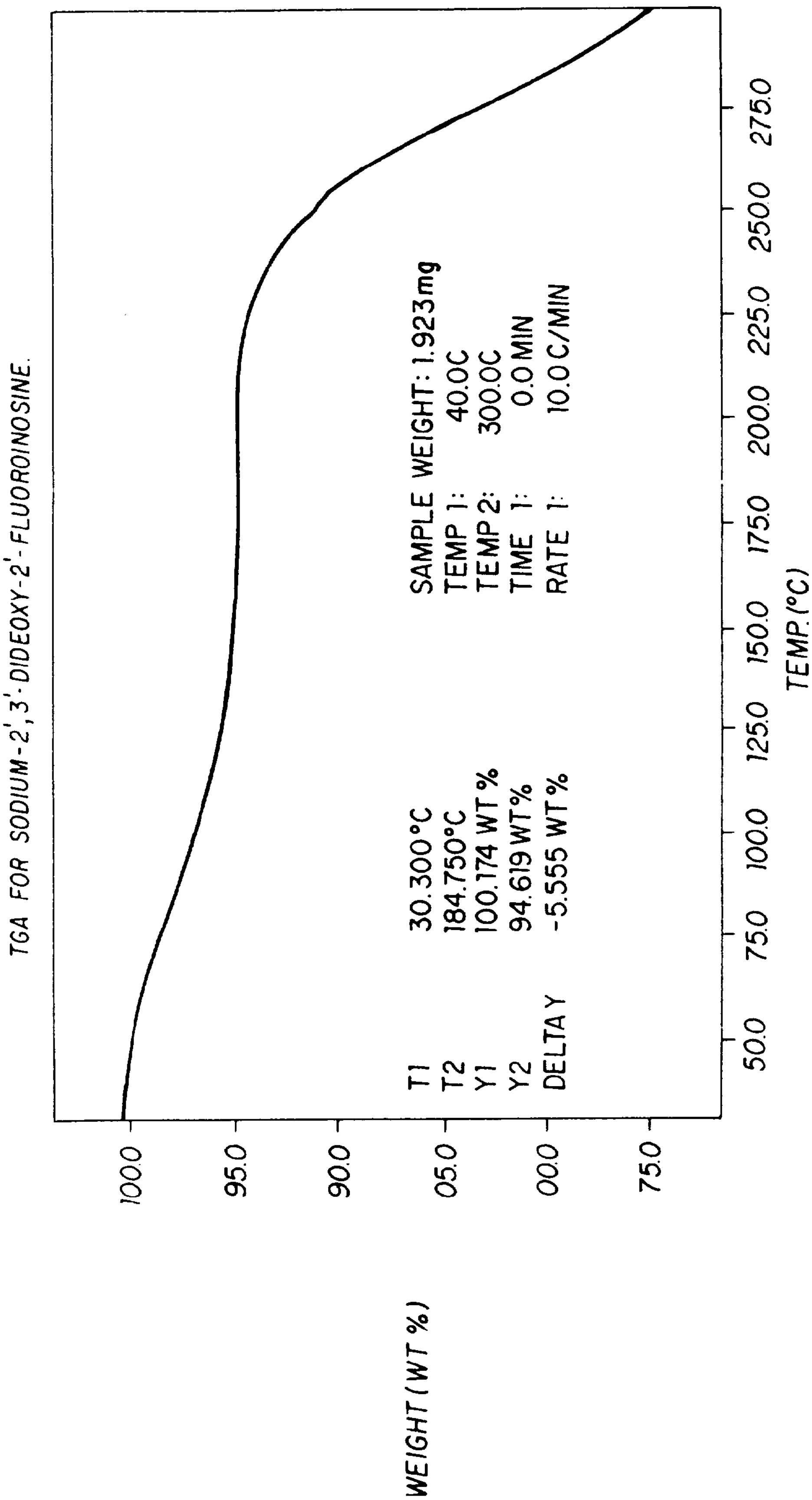
TGA FOR SODIUM-2',3'-DIDEOXY-2'-FLUOROINOSINE.
WEIGHT (WT %)
100.0
95.0
90.0
05.0
00.0
75.0
TEMP. (°C)
50.0
75.0
100.0
125.0
150.0
175.0
200.0
225.0
250.0
275.0
T1 30.300°C
T2 184.750°C
Y1 100.174 WT %
Y2 94.619 WT %
DELTA Y -5.555 WT %
SAMPLE WEIGHT: 1.923mg
TEMP 1: 40.0C
TEMP 2: 300.0C
TIME 1: 0.0 MIN
RATE 1: 10.0 C/MIN

FIG. 13